# FASCICULE DE BREVET EUROPEEN

(11) **EP 1 555 009 B1**
(45) Date de publication et mention de la délivrance du brevet: **14.07.2010**
(21) Numéro de dépôt: 04292991.9
(22) Date de dépôt: 14.12.2004
(51) Int. Cl.: A61K 8/19, A61K 8/89, A61K 8/34, A61K 8/31, A61K 8/92, A61Q 1/02, A61Q 1/08, A61Q 1/10, A61Q 1/12

(54) **Composition de maquillage de la peau**
Hautschminkezusammensetzung
Skin make-up composition

(30) Priorité: 05.01.2004 FR 0450008
(43) Date de publication de la demande: 20.07.2005
(73) Titulaire: L'Oréal, 75008 Paris (FR)
(72) Inventeur: Hadasch, Anke, 75013 Paris (FR); Delacour, Marie-Laure, 75013 Paris (FR); Mori, Isabelle, 92410 Ville d'Avray (FR)
(74) Mandataire: Boulard, Denis

(56) Documents cités:
- EP-A- 0 998 901
- EP-A- 1 023 893
- EP-A- 1 040 816
- EP-A- 1 099 437
- EP-A- 1 195 155
- WO-A-99/13823
- WO-A-02/053126

## Description

La présente invention a pour objet une composition de maquillage comprenant des particules solides et des matières colorantes particulières. L'invention a également pour objet un procédé de maquillage de la peau ou des lèvres d'être humain comprenant l'application de la composition sur la peau ou les lèvres.

La composition de maquillage peut être un produit de maquillage de la peau, tel qu'un produit du teint (notamment fond de teint), un fard à paupières, un fard à joue, un eye-liner, un produit anticemes, un produit de maquillage du corps, un produit de maquillage des lèvres. De préférence, la composition est une composition de maquillage de la peau, en particulier un fond de teint, un fard à paupières, un fard à joues. Plus spécialement, l'invention porte sur une composition de maquillage du teint, notamment un fond de teint, et sur une composition de fard à paupières.

Les produits de maquillage comme les fards à paupières comprennent généralement une phase pulvérulente contenant notamment des charges et des pigments. Or certaines charges comme le talc, de part leur opacité à la lumière, ont tendance à diminuer la saturation de la couleur des pigments présents dans la composition et il en résulte que le maquillage obtenu présente une couleur apparente moins vive que la couleur des matières colorantes seules. L'atténuation de la couleur du maquillage est notamment bien perceptible pour des fards à paupières sous forme de poudres (poudres libres ou poudres compactes) dont la teneur en charges est élevée, par exemple d'au moins 50 % en poids.

Il est aussi connu l'emploi comme matière colorante de particules réfléchissantes comme les particules de verre enrobées de métal comme décrit dans la demande ; EP-A-1082952 ou bien encore des pigments goniochromatiques à structure multicouches interférentielles ou à structure cristaux liquides comme décrites dans la demande EP-A-953330. Ces matières colorantes produisent des effets optiques colorés originaux, intenses, et différents de ceux obtenus avec les pigments classiques comme les oxydes de fer ou le dioxyde de titane. Or si ces matières colorantes originales sont formulées avec des charges opaques telles que le talc, en particulier lorsqu'elles sont présentes en quantité importante, ces charges opaques vont atténuer l'effet coloré original du produit et le maquillage obtenu présente alors un effet coloré diminué. Ainsi, l'intensité des effets optiques des matières colorantes est masquée par l'opacité de ces charges. Il faut alors introduire une grande quantité de matières colorantes pour que les effets colorés (effet optiques) recherchés soient bien visibles.

Il est connu du document WO 99/13823 des compositions de soin des cheveux comprenant un azurant optique et un agent de conditionnement des cheveux choisi parmi les composés siliconés, les composés cationiques, les composés ayant un haut point de fusion, les parfums, les huiles à haut poids moléculaire insolubles dans l'eau, et leur mélange. Il est également connu du document EP-A-988901 des compositions cosmétiques comprenant un composé photochrome constitué de mica enrobé de dioxyde de titane.

Pour les produits de maquillage se présentant sous une forme différente de celle d'une poudre, comme par exemple une crème, un gel, un produit coulé, qu'il soit notamment anhydre ou aqueux, les matières pulvérulentes ne peuvent être intro duites généralement que jusqu'à un taux de 30 % en poids. Aussi, lorsque ces compositions comprennent des charges opaques comme le talc, il n'est pas possible d'introduire de grande quantité de matière colorantes à effet optique telles que celles citées précédemment, limitant ainsi la possibilité d'obtenir un maquillage présentant un effet coloré intense.

Le but de la présente invention est donc de disposer d'une composition de maquillage contenant des matières colorantes et présentant un effet coloré peu atténué par rapport à l'effet coloré des matières colorantes seules.

Les inventeurs ont découvert qu'une telle composition est obtenue en utilisant des matières colorantes spécifiques, associées à un liant liquide et à des particules solides particuliers.

De façon plus précise, l'invention a pour objet une composition de maquillage poudreuse comprenant des particules solides d'un matériau ayant un indice de réfraction allant de 1,2 à 1,45, lesdites particules se présentant sous la forme d'une dispersion aqueuse, un liant liquide ayant un indice de réfraction allant de 1,2 à 1,6, et une matière colorante choisie parmi les particules réfléchissantes différentes des particules réfléchissantes comprenant un substrat de mica, les agents de coloration goniochromatiques, les agents de coloration photochromes, les agents fluorescents, et leurs mélanges.

L'invention a également pour objet un procédé cosmétique de maquillage de la peau ou des lèvres comprenant l'application sur la peau ou sur les lèvres d'une composition telle que définie précédemment. Le procédé est de préférence destiné à la peau.

La composition selon l'invention permet d'obtenir un maquillage présentant un effet coloré intense, peu diminué par rapport à l'effet coloré des matières colorantes seules. En particulier, le choix sélectif des particules solides et du liant liquide permet de "booster" l'effet coloré (effet optique) des matières colorantes spécifiques utilisées, procurant ainsi un maquillage intense, éclatant.

La composition selon l'invention comprend des particules solides d'organopolysiloxane réticulé élastomère ayant un indice de réfraction allant de 1,2 à 1,45 (notamment de 1,2 à 1,43). De préférence, l'indice de réfraction de ces particules va de 1,25 à 1,45 (notamment de 1,25 à 1,43), de préférence va de 1,3 à 1,45 (notamment de 1,3 à 1,43), préférentiellement va de 1,35 à 1,45 (notamment de 1,35 à 1,43), plus préférentiellement va de 1,38 à 1,45 (notamment va de 1,38 à 1,43), et encore plus préférentiellement va de 1,40 à 1,45 (notamment va de 1,40 à 1,43).

On entend par particules solides des particules de matériau étant à l'état solide (donc non liquide) à la température ambiante (25 °C).

L'indice de réfraction d'une particule solide est déterminée en effectuant un mélange de ladite particule solide en une teneur allant d'environ 1 % à 10 % en poids du poids total du mélange avec un milieu liquide comprenant une huile ou un solvant ou un mélange d'huiles ou de solvants choisi(e) de telle sorte que le mélange particules + milieu liquide soit transparent, c'est à dire que ce mélange soumis à un rayonnement dans la gamme de longueur d'onde 400-700 nm à l'aide d'un spectrophotomètre présente une transmission directe est égale à 100 %.

L'indice de réfraction de la particule est alors égal à l'indice de réfraction du milieu permettant d'obtenir une transmission directe égale à 100 %.
Si le milieu liquide contient une seule huile ou un seul solvant, l'indice de réfraction de la particule solide est égal à l'indice de réfraction de l'huile ou du solvant.
Si le milieu liquide contient un mélange d'huiles ou de solvants, l'indice de réfraction est calculé en fonction de la proportion volumique de chaque huile ou solvant dans le milieu liquide pondéré par leur indice de réfraction. On peut par exemple utiliser un mélange d'éthanol (qui a un indice de réfraction égal à 1,36) et d'alcool phénylique (qui a un indice de réfraction égal à 1,529).
La détermination de l'indice de réfraction est faite à la température ambiante (25 °C).

Par organopolysiloxane « élastomère » on entend un organopolysiloxane souple, déformable ayant des propriétés viscoélastiques et notamment la consistance d'une éponge ou d'une sphère souple. Son module d'élasticité est tel que ce matériau résiste à la déformation et possède une capacité limitée à l'extension et à la contraction. Ce matériau est capable de retrouver sa forme originelle suite à un étirement.

L'organopolysiloxane réticulé élastomère peut être obtenu par réaction d'addition réticulation de diorganopolysiloxane contenant au moins un hydrogène lié au silicium et de diorganopolysiloxane ayant des groupements à insaturation éthylénique liés au silicium, notamment en présence de catalyseur platine ; ou par réaction de condensation réticulation déhydrogénation entre un diorganopolysiloxane à terminaisons hydroxyle et un diorganopolysiloxane contenant au moins un hydrogène lié au silicium, notamment en présence d'un organoétain ; ou par réaction de condensation réticulation d'un diorganopolysiloxane à terminaisons hydroxyle et d'un organopolysilane hydrolysable ; ou par réticulation thermique d'oranopolysiloxane, notamment en présence de catalyseur organopéroxyde ; ou par réticulation d'organopolysiloxane par radiations de haute énergie telles que rayons gamma, rayons ultraviolet, faisceau électronique.

De préférence, l'organopolysiloxane réticulé élastomère est obtenu par réaction d'addition réticulation (A) de diorganopolysiloxane contenant au moins deux hydrogènes liés chacun à un silicium, et (B) de diorganopolysiloxane ayant au moins deux groupements à insaturation éthylénique liés au silicium, notamment en présence (C) de catalyseur platine, comme par exemple décrit dans la demande EP-A-295886.

En particulier, l'organopolysiloxane peut être obtenu par réaction de diméthylpolysiloxane à terminaisons diméthylvinylsiloxy et de méthylhydrogénopolysiloxane à terminaisons triméthylsiloxy, en présence de catalyseur platine.

Le composé (A) est le réactif de base pour la formation d'organopolysiloxane élastomère et la réticulation s'effectue par réaction d'addition du composé (A) avec le composé (B) en présence du catalyseur (C).

Le composé (A) est en particulier un organopolysiloxane ayant au moins 2 atomes d'hydrogène liés à des atomes de silicium distincts dans chaque molécule.

Le composé (A) peut présenter toute structure moléculaire, notamment une structure chaîne linéaire ou chaîne ramifiée ou une structure cyclique.

Le composé (A) peut avoir une viscosité à 25 °C allant de 1 à 50 000 centistokes, notamment pour être bien miscible avec le composé (B).

Les groupes organiques liés aux atomes de silicium du composé (A) peuvent être des groupes alkyles tels que méthyl, éthyl, propyl, butyl, octyl ; des groupes alkyles substitués tels que 2-phényléthyl, 2-phénylpropyl, 3,3,3-trifluoropropyl ; des groupes aryles tels que phényl, tolyl, xylyl ; des groupes aryles substitués tels que phényléthyl ; et des groupes hydrocarbonés monovalents substitués tels qu'un groupe époxy, un groupe ester carboxylate, ou un groupe mercapto.

Le composé (A) peut ainsi être choisi parmi les méthylhydrogénopolysiloxanes à terminaisons triméthylsiloxy, les copolymères diméthylsiloxane-méthylhydrogénosiloxane à terminaisons triméthylsiloxy, les copolymères cycliques diméthylsiloxane-méthylhydrogénosiloxane.

Le composé (B) est avantageusement un diorganopolysiloxane ayant au moins deux groupes alkényles inférieurs (par exemple en C₂-C₄) ; le groupe alkényle inférieur peut être choisi parmi les groupes vinyl, allyl, et propényl. Ces groupements alkényles inférieurs peuvent être situés en toute position de la molécule organopolysiloxane mais sont de préférence situés aux extrémités de la molécule organopolysiloxane. L'organopolysiloxane (B) peut avoir une structure à chaîne ramifiée, à chaîne linéaire, cyclique ou en réseau mais la structure en chaîne linéaire est préférée. Le composé (B) peut avoir une viscosité allant de l'état liquide à l'état de gomme. De préférence, le composé (B) a une viscosité d'au moins 100 centistokes à 25 °C.

Outre les groupes alkényle précités, les autres groupes organiques liés aux atomes de silicium dans le composé (B) peuvent être des groupes alkyles tels que méthyl, éthyl, propyl, butyl ou octyl ; des groupes alkyles substitués tels que 2-phényléthyl, 2-phénylpropyl ou 3,3,3-trifluoropropyl ; des groupes aryles tels que phényl, tolyl ou xylyl ; des groupes aryles substitués tels que phényléthyl ; et des groupes hydrocarbonés monovalents substitués tels qu'un groupe époxy, un groupe ester carboxylate, ou un groupe mercapto.

Les organopolysiloxanes (B) peuvent être choisis parmi les méthylvinylpolysiloxanes, les copolymères méthylvinylsiloxane-diméthylsiloxane, les diméthylpolysiloxanes à terminaisons diméthylvinylsiloxy, les copolymères diméthylsiloxaneméthylphénylsiloxane à terminaisons diméthylvinylsiloxy, les copolymères diméthylsiloxane-diphénylsiloxane-méthylvinylsiloxane à terminaisons diméthylvinylsiloxy, les copolymères diméthylsiloxane-méthylvinylsiloxane à terminaisons triméthylsiloxy, les copolymères diméthylsiloxane-méthylphénylsiloxaneméthylvinylsiloxane à terminaisons triméthylsiloxy, les méthyl(3,3,3-trifluoropropyl)polysiloxane à terminaisons diméthylvinylsiloxy, et les copolymères diméthylsiloxane-méthyl(3,3,3-trifluoropropyl)siloxane à terminaisons diméthylvinylsiloxy.

En particulier, l'organopolysiloxane élastomère peut être obtenu par réaction de diméthylpolysiloxane à terminaisons diméthylvinylsiloxy et de méthylhydrogénopolysiloxane à terminaisons triméthylsiloxy, en présence de catalyseur platine.

Avantageusement, la somme du nombre de groupements éthyléniques par molécule du composé (B) et du nombre d'atomes d'hydrogène liés à des atomes de silicium par molécule du composé (A) est d'au moins 5.

Il est avantageux que le composé (A) soit ajouté en une quantité telle que le rapport moléculaire entre la quantité totale d'atomes d'hydrogène liés à des atomes de silicium dans le composé (A) et la quantité totale de tous les groupements à insaturation éthylénique dans le composé (B) soit compris dans la gamme de 1,5/1 à 20/1.

Le composé (C) est le catalyseur de la réaction de réticulation , et est notamment l'acide chloroplatinique, les complexes acide chloroplatinique-oléfine, les complexes acide chloroplatinique-alkenylsiloxane, les complexes acide chloroplatinique-dicétone, le platine noir, et le platine sur support.

Le catalyseur (C) est de préférence ajouté de 0,1 à 1000 parts en poids, mieux de 1 à 100 parts en poids, en tant que métal platine propre pour 1000 parts en poids de la quantité totale des composés (A) et (B).

L'élastomère est avantageusement un élastomère non-émulsionnant.

Le terme "non émulsionnant" défini des élastomères organopolysiloxane ne contenant pas de motifs polyoxyalkylène (notamment polyoxyéthylène ou polyoxypropylène).

En particulier, les particules d'organopolysiloxane élastomérique (en matière active) peuvent avoir une taille moyenne allant de 0,1 à 500 µm, de préférence de 3 à 200 µm et mieux de 10 et 20 µm. Ces particules peuvent être de forme sphérique, plate ou amorphe, et de préférence de forme sphérique.

Selon l'invention, les particules d'organopolysiloxane réticulé élastomère utilisées sont sous la forme d'une dispersion aqueuse, telle que décrite dans la demande JP-A-10/175816 ou le brevet US 5928660. Selon cette demande, l'organopolysiloxane réticulé élastomère est obtenu par réaction d'addition et de réticulation, en présence d'un catalyseur en particulier du type platine, d'au moins :
- (a) un organopolysiloxane (i) ayant au moins deux groupes vinyliques en position α-ω de la chaîne siliconée par molécule ; et
- (b) un organosiloxane (ii) ayant au moins un atome d'hydrogène lié à un atome de silicium par molécule.

La dispersion aqueuse peut être notamment obtenue comme suit :
- (a) mélange d'un organopolysiloxane (i) ayant au moins deux groupes vinyliques en position α-ω de la chaîne siliconée par molécule et d'un organosiloxane (ii) ayant au moins un atome d'hydrogène lié à un atome de silicium par molécule ;
- (b) ajout d'un catalyseur, en particulier de type platine ;
- (b) ajout d'une phase aqueuse contenant un émulsifiant pour former une émulsion ;
- (c) polymérisation de l'organopolysiloxane (i) et de l'organosiloxane (ii) en émulsion en présence d'un catalyseur de platine.

En particulier, l'organopolysiloxane (i) est choisi parmi les n α-ω-diméthylvinyl polydiméthylsiloxanes.

L'émulsionnant peut être choisis parmi les tensioactifs non ioniques, cationiques ou anioniques de HLB ≥ 8, de préférence choisi parmi les tensioactifs non ioniques.
La proportion de tensioactifs est de préférence de 0,1 à 20 parties en poids pour 100 parties en poids de la composition d'organopolysiloxane élastomérique, et mieux, de 0,5 à 10 parties en poids (cf. description du document JP-A-10/175816).

Après l'étape (c), il est possible de sécher les particules obtenues, pour en évaporer toute ou partie de l'eau piégée.

Comme particules d'organopolysiloxane en dispersion dans l'eau, on peut utiliser celles commercialisées sous les noms BY 29-122 et BY 29-119 par la société Dow-Corning.

Les particules solides d'organopolysiloxane réticulé élastomère ayant un indice de réfraction allant de 1,2 à 1,45 peuvent être présentes dans la composition selon l'invention en une teneur allant de 5 % à 80 % en poids, par rapport au poids total de la composition, de préférence allant de 10 % à 70 % en poids, de préférence allant de 10 % à 60 % en poids, préférentiellement allant de 10 % à 50 % en poids, plus préférentiellement allant de 15 % à 50 % en poids, plus préférentiellement allant de 20 % à 45 % en poids, et encore plus préférentiellement allant de 20 % à 40 % en poids.

Le liant liquide présent dans la composition selon l'invention est un liquide ayant un indice de réfraction allant de 1,2 à 1,6, de préférence allant de 1,2 à 1,5, préférentiellement allant de 1,25 à 1,45, et plus préférentiellement allant de 1,3 à 1,45.

Le liant liquide peut être choisi parmi l'eau, les solvants organiques miscibles à l'eau, les huiles, et leurs mélanges ; et de préférence choisi parmi l'eau et les solvants organiques miscibles à l'eau, et leurs mélanges.

L'eau peut être une eau florale telle que l'eau de bleuet et/ou une eau minérale telle que l'eau de VITTEL, l'eau de LUCAS ou l'eau de LA ROCHE POSAY et/ou une eau thermale.

La composition peut comprendre de l'eau en une teneur allant de 5 % à 30 % en poids, par rapport au poids total de la composition, de préférence allant de 10 % à 25 % en poids, préférentiellement allant de 15 % à 25 % en poids, et plus préférentiellement allant de 20 % à 25 % en poids.

Les solvants organiques miscibles à l'eau à la température ambiante (25 °C) peuvent être par exemple les monoalcools ayant de 2 à 6 atomes de carbone tels que l'éthanol, l'isopropanol ;
les polyols ayant notamment de 2 à 20 atomes de carbones, de préférence ayant de 2 à 10 atomes de carbone, et préférentiellement ayant de 2 à 6 atomes de carbones, tels que la glycérine, le propylène glycol, le butylène glycol, le pentylène glycol, l'hexylène glycol, le dipropylène glycol, le diéthylène glycol ;
les éthers de glycol (ayant notamment de 3 à 16 atomes de carbone) tels que les alkyl(C₁-C₄)éther de mono, di- ou tripropylène glycol, les alkyl(C₁-C₄)éthers de mono, di- ou triéthylène glycol ;
et leurs mélanges.

La composition selon l'invention peut comprendre un solvant organique miscible à l'eau, notamment un polyol, en une teneur allant de 1 % à 20 % en poids, par rapport au poids total de la composition, et de préférence allant de 3 % à 15 % en poids.

Les huiles peuvent être choisies parmi les huiles hydrocarbonées, les huiles siliconées, les huiles fluorées, et leurs mélanges. L'huile peut être notamment choisies parmi les huiles volatiles.

Par huile hydrocarbonée, on entend une huile formée essentiellement, voire constituée, d'atomes de carbone et d'hydrogène, et éventuellement d'atomes d'oxygène, d'azote, et ne contenant pas d'atome de silicium ou de fluor. Elle peut contenir des groupes alcool, ester, éther, acide carboxylique, amine et/ou amide.

Par "huile volatile", on entend tout milieu non aqueux non miscible à l'eau susceptible de s'évaporer de la peau ou des lèvres, en moins d'une heure, et notamment les huiles ayant une pression de vapeur, à température ambiante et pression atmosphérique allant de 10⁻³ à 300 mm de Hg (0,13 Pa à 40 000 Pa).

Les huiles hydrocarbonées peuvent être par exemple l'huile de paraffine ou de vaseline, l'huile de vison, de tortue, de soja, le squalane, le perhydrosqualène, l'huile d'amande douce, de calophyllum, de palme, de pépins de raisin, de sésame, de maïs, de polyisobutylène hydrogéné (parléam), d'arara, de colza, de tournesol, de coton, d'abricot, de ricin, d'avocat, de jojoba, d'olive ou de germes de céréales ; les esters d'acide lanolique, d'acide oléique, d'acide laurique, d'acide stéarique ; les esters gras tels que le myristate d'isopropyle, le palmitate d'isopropyle, le stéarate de butyle, le laurate d'hexyle, l'adipate de diisopropyle, l'isononate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de 2-diéthyl-hexyle, le malate de diisostéaryle, le triisostéarate de glycérine ou de diglycérine ; les acides gras supérieurs tels que l'acide myristique, l'acide palmitique, l'acide stéarique, l'acide béhénique, l'acide oléique, l'acide linoléique, l'acide linolénique ou l'acide isostéarique ; les alcools gras supérieurs tels que le cétanol ou l'alcool oléique, l'alcool linoléique ou linolénique, l'alcool isostéarique ou l'octyl dodécanol ;

Les huiles siliconées peuvent être par exemple les polydiméthylsiloxanes (PDMS), éventuellement phénylées telles que les phényltriméthicones ou éventuellement substitués par des groupements aliphatiques et/ou aromatiques, éventuellement fluorés, ou par des groupements fonctionnels tels que des groupements hydroxyle, thiol et/ou amine ; les polysiloxanes modifiés par des acides gras, des alcools gras ou des polyoxyalkylènes, les silicones fluorées.

Avantageusement, on peut utiliser une ou plusieurs huiles volatiles à température ambiante. Elles peuvent être hydrocarbonées ou siliconées et comporter éventuellement des groupements alkyle ou alkoxy, pendants ou en bout de chaîne siliconée.

Comme huile volatile utilisable dans l'invention, on peut citer les silicones linéaires ou cycliques ayant de 2 à 7 atomes de silicium, ces silicones comportant éventuellement des groupes alkyle ou alkoxy ayant de 1 à 10 atomes de carbone notamment l'octaméthylcyclotétrasiloxane, le décaméthylcyclopentasiloxane, l'hexadécaméthylcyclohexasiloxane, l'heptaméthylhexyltrisiloxane et l'heptaméthyloctyltrisiloxane, ainsi que les isoparaffines en C₈-C₁₆ telles que les ISOPARs®, les PER-METYLs® et notamment l'isododécane.

Le liant liquide de la composition peut être présent dans la composition selon l'invention en une teneur allant de 5 % à 60 % en poids, par rapport au poids total de la composition, de préférence allant de 5 % à 50 % en poids, de préférence allant de 5 % à 45 % en poids, préférentiellement allant de 10 % à 40 % en poids, et plus préférentiellement allant de 15 % à 35 % en poids.

La matière colorante présente dans la composition est choisie parmi les particules réfléchissantes différentes des particules réfléchissantes comprenant un substrat de mica, les agents de coloration goniochromatiques, les agents de coloration photochromes, les agents fluorescents, et leurs mélanges.

La composition peut comprendre comme matière colorante des particules réfléchissantes différentes des particules réfléchissantes comprenant un substrat de mica.

Les particules réfléchissantes peuvent être choisies parmi :
- les particules à substrat naturel ou synthétique enrobé au moins partiellement par au moins une couche d'au moins un métal,
- les particules à substrat synthétique, différent du mica, enrobé au moins partiellement par au moins une couche d'au moins un composé métallique et notamment d'un oxyde métallique,
- les particules formées d'un empilement d'au moins deux couches de matériaux à indices de réfraction différents, l'une au moins de ces couches pouvant être un polymère, et
- les particules métalliques ;
- et leurs mélanges.

Par « particules réfléchissantes », on désigne au sens de la présente invention des particules dont la taille, la structure, notamment l'épaisseur de la ou des couches qui la constituent et leur natures physique et chimique, et l'état de surface, leur permettent de réfléchir la lumière incidente avec une intensité suffisante pour pouvoir créer à la surface de la composition revendiquée, lorsque cette dernière est appliquée sur le support à maquiller, des points de brillance visibles à l'oeil nu, c'est-à-dire des points plus lumineux qui contrastent avec leur environnement en semblant briller.
Les particules réfléchissantes peuvent troubler la perception visuelle de la courbure du support maquillé, en tendant à empêcher une focalisation visuelle durable, les points de brillance étant susceptibles d'apparaître ou de disparaître de manière aléatoire lorsque le support maquillé et l'observateur sont animés.

Les particules réfléchissantes utilisées doivent être compatibles avec une utilisation en cosmétique et doivent pouvoir subsister dans la composition, et notamment ne pas se dissoudre, ou en tout cas ne pas se dissoudre entièrement, dans celle-ci.

Les particules réfléchissantes ont avantageusement une taille compatible avec la manifestation d'une réflexion spéculaire de la lumière visible (400-700 nm), d'intensité suffisante pour créer un point de brillance. Cette taille est susceptible de varier selon la nature chimique des particules, leur forme et leur pouvoir de réflexion spéculaire de la lumière visible.

Parmi les particules réfléchissantes utilisables dans l'invention, certaines peuvent présenter un écart relatif Δ, défini par la formule Δ= [L *_{SCI} - L*_{SCE]} / L *_{SCE}, supérieur ou égal à 0,25. Par comparaison, certaines nacres qui ne conviennent pas en tant que particules réfléchissantes présentent un coefficient Δ inférieur à 0,25. Dans la formule ci-dessus, L *_{SCI} désigne la clarté L* mesurée à l'aire d'un spectrocolorimètre de marque MINOLTA et de référence CM-2002, dans un mode dit « composante spéculaire incluse », et L*_{SCE}, la clarté L* mesurée à l'aide du même appareil, dans un mode dit « composante spéculaire exclue ». Pour effectuer les mesures, on réalise une dispersion à 5 % en poids des particules à tester dans un vernis à ongles transparent de composition classique (essentiellement de la nitrocellulose, une résine et un plastifiant) et l'on étale à l'état fluide une couche de 300 µm d'épaisseur de la composition ainsi formée sur le fond noir d'une carte de contraste.
On utilise la fonction SCI/SCE du spectrocolorimètre avec la géométrie d/8 pour mesurer L*_{SCI} et L*_{SCE}.
A titre d'exemple, on a mesuré pour des particules réfléchissantes de marque REFLECKS®, commercialisées par la société ENGELHARD, comportant un substrat de verre enrobé d'oxyde de fer brun, un écart relatif Δ supérieur à 0,7 .
Les particules réfléchissantes peuvent présenter des formes variées. Ces particules peuvent être notamment en forme de plaquettes ou globulaires, en particulier sphériques.
Par l'expression « en forme de plaquette », on désigne des particules dont le rapport de la plus grande dimension à l'épaisseur est supérieur ou égal à 5, voire 10 ou mieux 20. L'épaisseur des particules en forme de plaquettes est par exemple comprise entre environ 0,5 µm et environ 5 µm.

Les particules présentant une surface extérieure sensiblement plane conviennent tout particulièrement, car elles peuvent plus facilement donner naissance, si leur taille, leur structure et leur état de surface le permet, à une réflexion spéculaire intense. On parle d'effet miroir.
Pour de telles particules notamment, c'est essentiellement la lumière renvoyée par réflexion dans une direction faisant, avec la normale à la surface réfléchissante, le même angle que celui que fait la lumière incidente avec cette normale, qui permet à ces particules d'apparaître comme des points de brillance, et non la lumière diffusée dans les autres directions.

Il peut être souhaitable que les particules réfléchissantes soient non diffusantes et non mates.
Il peut être souhaitable également que les particules réfléchissantes n'altèrent pas de manière sensible la coloration de la composition cosmétique.
A cet égard, les particules réfléchissantes qui permettent une réflexion métallique de la lumière incidente conviennent tout particulièrement. C'est le cas notamment lorsque les particules réfléchissantes permettent quelque soit leur forme, une réflexion sur une couche d'un métal, par exemple de l'argent. De telles particules s'avèrent relativement neutres vis-à-vis de la couleur de la composition.

Des particules réfléchissantes utilisables dans l'invention, à reflet métallique ou blanc, peuvent par exemple réfléchir la lumière dans toutes les composantes du visible sans absorber de manière significative une ou plusieurs longueurs d'ondes. La réflectance spectrale de ces particules réfléchissantes peut par exemple être supérieure à 70 % dans l'intervalle 400-700 nm, et mieux d'au moins 80 %, voire 90 % ou encore 95 %.

La lumière réfléchie par les particules réfléchissantes peut être non iridescente, notamment dans le cas d'un reflet métallique.

Les particules réfléchissantes quelque soit leur forme, peuvent présenter une structure multicouche ou non et, dans le cas d'une structure multicouche, par exemple au moins une couche d'épaisseur uniforme, notamment d'un matériau réfléchissant.

Lorsque les particules réfléchissantes présentent une structure multicouche, celles-ci peuvent par exemple comporter un substrat naturel ou synthétique, notamment un substrat synthétique au moins partiellement enrobé par au moins une couche d'un matériau réfléchissant.

Quelle que soit la forme des particules réfléchissantes, le substrat peut, lorsqu'il est synthétique, être réalisé avec une forme favorisant la formation d'une surface réfléchissante après revêtement, notamment après dépôt d'une couche de matériau réfléchissant. Le substrat peut, par exemple, présenter une surface plane et la couche de matériau réfléchissant une épaisseur sensiblement uniforme.
Le substrat peut être monomatière ou multimatériaux, plein ou creux. Le substrat peut être organique ou inorganique. Le substrat peut être naturel mais de préférence on utilise un substrat synthétique, pour la raison indiquée ci-dessus.
Le substrat peut être choisi par les verres, les céramiques, le graphite, les oxydes métalliques, les alumines, les silices, les silicates, notamment les aluminosilicates et les borosilicates et le mica synthétique, cette liste n'étant pas limitative.

Le matériau réfléchissant peut comporter une couche de métal ou d'un composé métallique tel qu'un oxyde métallique.
La couche de métal ou de composé métallique peut enrober ou non en totalité le substrat et la couche de métal peut être au moins partiellement recouverte par une couche d'un autre matériau, par exemple un matériau transparent. Il peut être préférable que la couche de métal ou de composé métallique enrobe en totalité, directement ou indirectement, c'est-à-dire avec interposition d'au moins une couche intermédiaire, métallique ou non, le substrat.
Le métal peut être choisi par exemple parmi Ag, Au, Cu, Al, Ni, Sn, Mg, Cr, Mo, Ti, Pt, Va, Rb, W, Zn, Ge, Te, Se et leurs alliages. Ag, Au, Al, Zn, Ni, Mo, Cr, Cu et leurs alliages (par exemple les bronzes et les laitons) sont des métaux préférés.
Dans le cas notamment de particules à substrat enrobé d'argent ou d'or, la couche métallique peut être présente à une teneur représentant par exemple de 0,1 à 50 % du poids total des particules, voire entre 1 et 20 %.

Des particules de verre recouvertes d'une couche métallique peuvent avoir une dimension allant par exemple de 10 µm à 300 µm, et mieux de 25 µm à 150 µm. Dans le cas où ces particules sont en forme de plaquettes, l'épaisseur peut être comprise par exemple entre environ 0,1 µm et environ 25 µm, de préférence d'environ 0,5 µm à environ 10 µm et mieux d'environ 0,5 µm à environ 5 µm. Dans le cas où ces particules se trouvent sous forme de sphères, elles peuvent avoir une dimension allant par exemple d'environ 10 à 100 µm.

Des particules de verre recouvertes d'une couche métallique sont décrites notamment dans les documents JP-A-09188830, JP-A-10158450, JP-A-10158541, JP-A-07258460, JP-A-05017710, JP-A-2001-011340, JP-A-2002-138010, JP-A-2001-323217, JP-A-2001-207129.

Toujours à titre d'exemple de particules réfléchissantes comportant un substrat minéral enrobé d'une couche de métal, on peut citer encore les particules comportant un substrat de borosilicate enrobé d'argent.
Des particules à substrat de verre revêtu d'argent, en forme de plaquettes, sont vendues sous la dénomination MICROGLASS METASHINE REFSX 2025 PS par la société NIPPON SHEET GLASS. Des particules à substrat de verre revêtu d'alliage nickel/chrome/molybdène sont vendues sous la dénomination CRYSTAL STAR GF 550, GF 2525 par cette même société.

Les particules réfléchissantes quelque soit leur forme, peuvent également être choisies parmi les particules à substrat synthétique enrobé au moins partiellement d'au moins une couche d'au moins un composé métallique, notamment un oxyde métallique, choisi par exemple parmi les oxydes de titane, notamment TiO₂, de fer notamment Fe₂O₃, d'étain, de chrome, le sulfate de baryum et les composés suivants : MgF₂, CrF₃, ZnS, ZnSe, SiO₂, Al₂O₃, MgO, Y₂O₃, SeO₃, SiO, HfO₂, ZrO₂, CeO₂, Nb₂O₅, Ta₂O₅, MoS₂ et leurs mélanges ou alliages.
Des particules de verre recouvertes d'une couche d'oxyde métallique sont décrites notamment dans les documents JP-A-2001-207129, JP-A-2001-323217, US 3331699, US 5436077, EP-A-912640, WO 02/090448.
A titre d'exemple de telles particules, on peut citer par exemple les particules comportant un substrat de mica synthétique revêtu de dioxyde de titane, ou les particules de verre enrobé soit d'oxyde de fer brun, soit d'oxyde de titane, d'oxyde d'étain ou d'un de leurs mélanges comme celles vendues sous la marque REFLECKS® par la société ENGELHARD, ou "METASHINE 1080 R", "METASHINE MC 1120 RY", "METASHINE MC 1020 RS" par la société NIPPON SHEET GLASS.

Les particules réfléchissantes peuvent encore être choisies parmi les particules formées d'un empilement d'au moins deux couches à indices de réfraction différents.
Ces couches peuvent être de nature polymérique ou métallique et notamment inclure au moins une couche polymérique.
Ainsi, les particules réfléchissantes peuvent être des particules dérivant d'un film polymérique multicouche.
De telles particules sont notamment décrites dans WO 99/36477, US 6 299 979 et US 6 387 498.

A titre illustratif des matériaux pouvant constituer les différentes couches de la structure multicouche, on peut citer, cette liste n'étant pas limitative: le naphthalate de polyéthylène (PEN) et ses isomères par exemple 2,6-, 1,4-, 1,5-, 2,7- et 2,3-PEN, les téréphthalates de polyalkylene, des polyimides, des polyéthérimides, des polystyrènes atactiques, des polycarbonates, des polyméthacrylates et des polyacrylates d'alkyle, du polystyrène syndiotactique (sPS), des poly-alpha-méthylstyrène syndiotactiques, du polydichlorostyrène syndiotactique, copolymères et mélange de ses polystyrènes, des dérivés de cellulose, des polymères polyalkylènes, des polymères fluorés, des polymères chlorés, des polysulfones, des polyéthersulfones, des polyacrylonitriles, des polyamides, des résines siliconées, des résines époxy, de l'acétate de polyvinyle, des polyéthers-amides, des résines ionomériques, des élastomères et polyuréthanes. Conviennent également des copolymères, par exemple copolymères de PEN (par exemple, copolymères de 2,6-, 1,4-, 1,5-, 2,7-, et/ou 2,3-acide naphtalène dicarboxylique ou ses esters avec (a) acide téréphtalique ou ses esters ; (b) l'acide isophtalique ou ses esters ; (c) acide phtalique ou ses esters ; (d) des alcanes glycols ; (e) des cycloalkanes glycols (par exemple le cyclohexane diméthanol diol) ; (f) des acides alcanes dicarboxyliques ; et/ou (g) des acides cycloalkanes dicarboxylique, des copolymères de téréphthalates de polyalkylène et des copolymères de styrène. En outre, chaque couche individuelle peut inclure des mélanges de deux ou plusieurs polymères ou copolymères précédents.
Le choix des matériaux destinés à constituer les différentes couches de la structure multicouche est bien entendu effectué de manière à conférer l'aspect réfléchissant souhaité aux particules ainsi formées.
Des particules réfléchissantes comportant un empilement d'au moins deux couches de polymères sont commercialisées par la société 3M sous la dénomination MIRROR GLITTER. Ces particules comportent des couches de 2,6-PEN et de polyméthacrylate de méthyle dans un rapport massique de 80/20. De telles particules sont décrites dans le brevet US 5 825 643.
La brillance des particules réfléchissantes peut être encore due, en variante où additionnellement, à la réflexion de la lumière sur une couche d'un matériau de la particule présentant un indice de réfraction suffisant grand par rapport à celui du milieu d'où provient la lumière incidente.

Les particules métalliques réfléchissantes peuvent être des particules de métal tel que l'or, l'argent, l'aluminium, le cuivre.

La composition cosmétique selon l'invention peut bien entendu comporter des particules réfléchissantes de différentes natures sans que l'on sorte du cadre de la présente invention.

La matière colorante présente dans la composition selon l'invention peut être choisie parmi les agents de coloration goniochromatiques.

Par « agent de coloration goniochromatique », on désigne au sens de la présente invention un agent de coloration permettant d'obtenir, lorsque la composition le contenant est étalée sur un support, un trajet de couleur dans le plan a*b* de l'espace colorimétrique CIE 1976 qui correspond à une variation Dh de l'angle de la teinte h d'au moins 20° lorsque l'on fait varier l'angle d'observation par rapport à la normale entre 0° et 80°, pour un angle d'incidence de la lumière de 45°.

Le trajet de couleur peut être mesuré par exemple au moyen d'un spectrogonioréflectomètre de marque INSTRUMENT SYSTEMS et de référence GON 360 GONIOMETER, après que la composition cosmétique ait été étalée à l'état fluide avec une épaisseur de 300 µm au moyen d'un étaleur automatique sur une carte de contraste de marque ERICHSEN et de référence Typ 24/5, la mesure étant effectuée sur le fond noir de la carte.

L'agent de coloration goniochromatique peut être choisi de manière à présenter un changement de couleur relativement important avec l'angle d'observation.

L'agent de coloration goniochromatique peut être choisi par exemple parmi les structures multicouche interférentielles et les agents de coloration à cristaux liquides.

Dans le cas d'une structure multicouche, celle-ci peut comporter par exemple au moins deux couches, chaque couche, indépendamment ou non de la (ou les) autre(s) couche(s), étant réalisée par exemple à partir d'au moins un matériau choisi dans le groupe constitué par les matériaux suivants : MgF₂, CeF₃, ZnS, ZnSe, Si, SiO₂, Ge, Te, Fe₂O₃, Pt, Va, Al₂O₃, MgO, Y₂O₃, S₂O₃, SiO, HfO₂, ZrO₂, CeO₂, Nb₂O₅, Ta₂O₅, TiO₂, Ag, Al, Au, Cu, Rb, Ti, Ta, W, Zn, MoS₂, cryolithe, alliages, polymères et leurs associations.
Les agents goniochromatiques à structures multicouches sont notamment ceux décrits dans les documents suivants : US-A-3 438 796, EP-A-227423, US-A-5 135 812, EP-A-170439, EP-A-341002, US-A-4 930 866, US-A- 5 641 719, EP-A-472371, EP-A-395410, EP-A-753545, EP-A-768343, EP-A-571836, EP-A-708154, EP-A-579091, US-A-5 411 586, US-A- 5 364 467, WO-A-97/39066, DE-A-4 225 031, WO 9517479 (BASF), DE-A-196 14 637. Ils se présentent sous forme de paillettes, de couleur métallisée.

Les structures multicouches utilisables dans l'invention sont par exemple les structures suivantes : Al/SiO₂/Al/SiO₂/Al ; Cr/MgF₂/Al/MgF₂/Al ; MoS₂/SiO₂/Al/SiO₂/MoS₂ ; Fe₂O₃/SiO₂/Al/ SiO₂/Fe₂O₃ ; Fe₂O₃/SiO₂/Fe₂O₃/SiO₂/Fe₂O₃ ; MoS₂/SiO₂/mica-oxyde/SiO₂/MoS₂ ; Fe₂O₃/SiO₂/mica-oxyde/SiO₂/Fe₂O₃. Selon l'épaisseur des différentes couches, on obtient différentes couleurs. Ainsi, avec la structure Fe₂O₃/SiO₂/Al/ SiO₂/Fe₂O₃ on passe du doré-vert au gris-rouge pour des couches de SiO₂ de 320 à 350 nm ; du rouge au doré pour des couches de SiO₂ de 380 à 400 nm ; du violet au vert pour des couches de SiO₂ de 410 à 420 nm ; du cuivre au rouge pour des couches de SiO₂ de 430 à 440 nm.
Par suite la structure multicouche peut être essentiellement minérale ou organique. Selon l'épaisseur de chacune des différentes couches, on obtient différentes couleurs.

Les pigments goniochromatiques à structure multicouche interférentielle selon l'invention sont notamment ceux décrits dans les documents suivants : US-A-3 438 796, EP-A-227423, US-A-5 135 812, EP-A-170439, EP-A-341002, US-A-4 930 866, US-A- 5 641 719, EP-A-472371, EP-A-395410, EP-A-753545, EP-A-768343, EP-A-571836, EP-A-708154, EP-A-579091, US-A-5 411 586, US-A- 5 364 467, WO-A-97/39066, DE-A-4 225 031, WO 9517479 (BASF), DE-A-196 14 637, et leurs associations. Ils se présentent sous forme de paillettes, de couleur métallisée.

De préférence, le pigment goniochromatique à structure multicouche interférentielle selon l'invention est choisi dans le groupe constitué par les pigments goniochromatiques commerciaux suivants : Infinite Colors fabriqué ou commercialisé par la société SHISEIDO, Sicopearl Fantastico fabriqué ou commercialisé par la société BASF, Colorstream fabriqué ou commercialisé par la société MERCK, Colorglitter fabriqué ou commercialisé par la société 3M, Chromaflair fabriqué ou commercialisé par la société FLEX, Xirallic et Xirona fabriqués ou commercialisés par la société MERCK et leurs mélanges.

Comme agent goniochromatique à structure multicouche, on peut citer ceux vendus sous la dénomination "SICOPEARL

Quant aux particules goniochromatiques à cristaux liquides susceptibles d'être mises en oeuvre dans la composition selon l'invention, elles peuvent notamment être à base de polymère susceptible d'être obtenu par polymérisation d'un mélange de monomères comprenant :
- a) au moins un premier monomère A de formule (I) Y1-A1-M1-A2-Y2 dans laquelle
   - i) Y1 et Y2 , identiques ou différents, représentent un groupe polymérisable choisi parmi les groupements acrylate ou méthacrylate, un groupement époxy, un groupe isocyanate, hydroxy, vinyléther (-O-CH=CH₂) ou vinylester (-CO-O-CH=CH₂), et
   - ii) A1 et A2, identiques ou différents, représentent un groupement de formule
      -CnH₂n-, dans laquelle n est un nombre entier allant de 0 à 20 et un ou plusieurs groupes méthylène dudit groupement -CnH₂n- pouvant être remplacés par un ou plusieurs atomes d'oxygène, et
   - iii) M1 désigne un groupement de formule générale (l') -R₁-X₁-R₂-X₂-R₃-X₃-R₄-, dans laquelle R₁, R₂, R₃ et R₄ , identiques ou différents, désignent un groupement divalent choisi parmi -O-, -COO-, -CONH-, -CO-, -S-, -C≡C-, -CH=CH-, -N=N-, -N=N(O)-, et -R₂-X₂-R₃- ou -R₂-X₂- ou -R₂-X₂-R₃-X₃- pouvant également être une liaison covalente simple, et X₁,X₂,X₃ sont des groupements, identiques ou différents, choisis parmi les groupements 1,4-phénylène, 1,4-cyclohexylène, les groupements arylènes ou hétéroarylènes ayant un noyau aryle comprenant de 6 à 10 atomes éventuellement substitués par B1 et/ou B2 et/ou B3, ledit hétéroarylène contenant de 1 à 3 hétéroatomes choisis parmi les atomes O, N et S, les groupements cycloalkylènes ayant de 3 à 10 atomes de carbones éventuellement substitués par -B1 et/ou -B2 et/ou -B3, -B1, -B2, et -B3 , identiques ou différents, étant choisis parmi les groupes alkyle en C₁-C₂₀, alkoxy en C₁-C₂₀, alkylthio en C₁-C₂₀, alkyl(C₁-C₂₀) carbonyl, alkoxy(C₁-C₂₀) carbonyl, alkyl(C₁-C₂₀) thiocarbonyl, -OH, -F, -CI, -Br, -I, -CN, -NO₂, formyle, acétyle, et des groupements alkyle, alkoxy ou alkylthio ayant de 1 à 20 atomes de carbone, interrompus par un ou plusieurs atome(s) d'oxygène ou un ou plusieurs atome(s) de soufre ou un ou plusieurs groupe(s) ester,
      et
- b) au moins un deuxième monomère B chiral de formule (II) V1-A'1-W1-Z-W2-A'2-V2, dans laquelle
   - i) V1 et V2 , identiques ou différents, désignent un groupement choisi parmi un groupement acrylate ou méthacrylate, un groupement époxy, un groupement vinyléther ou vinylester, un groupement isocyanate, un alkyle en C₁-C₂₀, un alkoxy en C₁-C₂₀, un alkylthio en C₁-C₂₀, un alkoxy(C₁-C₂₀) carbonyl, un alkyl(C₁-C₂₀)thiocarbonyle, -OH, -F, -CI, -Br, -I, -CN, -NO2, formyle, acétyle et des groupements alkyle, alkoxy ou alkylthio, ayant de 1 à 20 atomes de carbone, et interrompus par un ou plusieurs atome(s) d'oxygène ou un ou plusieurs atome(s) de soufre ou un ou plusieurs groupe(s) ester (-CO-O-),
      et au moins V1 ou V2 désigne un groupe polymérisable choisi parmi les groupements acrylate ou méthacrylate, un groupement époxy, un groupe isocyanate, hydroxy, vinyléther (-O-CH=CH₂) ou vinylester (-CO-O-CH=CH₂),
   - ii) A'1 et A'2 , identiques ou différents, représentent un groupement de formule -CₙH₂ₙ-, dans laquelle n est un nombre entier allant de 0 à 20 et un ou plusieurs groupes méthylène dudit groupement CₙH₂ₙ pouvant être remplacés par un ou plusieurs atomes d'oxygène, et
   - iii) W1 et W2 désignent un groupement divalent de formule générale R'₁-X'₁-R'₂-X'₂-R'₃- dans laquelle R'₁ , R'₂ et R'₃, identiques ou différents, désignent un groupement divalent choisi parmi -O-, -COO-, -CONH-, -CO-, -S-, -C≡C-, -CH=CH-, -N=N-, -N=N(O)-, et R'₁, R'₂ , R'₃ ou R'₂-X'₂ peut également être une liaison covalente simple, et X'₁ et X'₂ sont des groupements, identiques ou différents, choisis parmi les groupements 1,4-phénylène, 1,4-cyclohexylène, les groupements arylènes ou hétéroarylènes ayant un noyau aryle comprenant de 6 à 10 atomes éventuellement substitués par B'1 et/ou B'2 et/ou B'3, ledit hétéroarylène contenant de 1 à 3 hétéroatomes choisis parmi les atomes O, N et S, les groupements cycloalkylènes ayant de 3 à 10 atomes de carbones éventuellement substitués par -B'1 et/ou -B'2 et/ou -B'3, -B'1, -B'2, et -B'3, identiques ou différents, étant choisis parmi les groupes alkyle en C₁-C₂₀, alkoxy en C₁-C₂₀, alkylthio en C₁-C₂₀, alkyl(C₁-C₂₀)carbonyl, alkoxy (C₁-C₂₀)carbonyl, alkyl(C₁-C₂₀)thiocarbonyl, -OH, -F, -CI, -Br, -I, -CN, -NO₂, formyle, acétyle, et des groupements alkyle, alkoxy ou alkylthio ayant de 1 à 20 atomes de carbone, interrompus par un ou plusieurs atome(s) d'oxygène ou un ou plusieurs atome(s) de soufre ou un ou plusieurs groupe(s) ester,
      et Z désigne un groupement chiral divalent comprenant au moins 4 atomes de carbone, notamment de 4 à 20 atomes de carbone, et mieux de 4 à 10 atomes de carbone, (le groupement chiral divalent comprenant au moins un carbone asymétrique, notamment un ou deux carbones asymétriques, et en particulier deux carbones asymétriques) et en particulier un groupe chiral divalent issu du groupe des dianhydrohexites, hexoses, pentoses, les dérivés binaphthyle (groupements binaphtyle), les dérivés biphényle (groupements biphényle), les dérivés d'acides tartriques ou des glycols optiquement actifs.

De préférence, le polymère à cristaux liquides est obtenu par polymérisation d'un mélange de monomères comprenant :
- a) au moins un premier monomère A de formule (I) Y1-A1-M1-A2-Y2
   dans laquelle
   - i) Y1 et Y2 , identiques ou différents, représentent un groupe acrylate ou méthacrylate, de préférence un groupe acrylate ;
   - ii) A1 et A2, identiques ou différents, représentent un groupement de formule
      -CnH₂n-, dans laquelle n est un nombre entier allant de 1 à 20, de préférence allant de 2 à 6, et mieux égal à 4 ;
   - iii) M1 désigne un groupement de formule générale (I') -R₁-X₁-R₂-X₂-R₃-X₃-R₄-, dans laquelle R1 et R4 désignent -O-, R₂ et R₃ désignent -COO-,
      et X1,X2,X3 sont un groupement 1,4 phénylène, le groupe carbonyl - CO- respectivement de R₂ et de R₃ étant lié respectivement au groupement X1, X3,
      et
- b) au moins un deuxième monomère B chiral de formule (II) V1-W1-Z-W2-V2, dans laquelle
   - i) V1 désigne un groupement acrylate ou méthacrylate, et de préférence un groupe acrylate, et V2 désigne un groupement alkyle en C₁-C₂₀, un alkoxy en C₁-C₂₀, un alkoxy(C₁-C₂₀)carbonyle, -OH, et de préférence désigne un groupement alkoxy en C₁-C₂₀, notamment en C₁-C₄, et en particulier un groupement méthoxy ;
   ii) W1 représente un groupement divalent de formule -X'1-CO-O-,
      W2 représente un groupement divalent de formule -O-CO-X'1-,
      dans lesquelles X'1 désigne un groupe 1,4-phénylène,
      et Z désigne un groupement chiral à deux liaisons, issu du groupement dianhydrohexite, en particulier un radical divalent de formule :

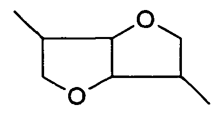

De préférence, le mélange de monomères comprend de 70 à 99 % en poids de monomère A et de 1 à 30 % en poids de monomère B, par rapport au poids total de monomère A et de monomère B, et mieux comprend de 90 à 95 % en poids de monomère A et de 5 à 10 % en poids de monomère B.

De préférence, la concentration des groupes polymérisables présents dans le mélange de monomère A et de monomère B (groupes polymérisables Y1, Y2 du monomère A et groupes polymérisables V1, V2 du monomère B) va de 3,2 à 15 mmoles/g.

Selon un mode particulier de réalisation de l'invention, le polymère à cristaux liquides est tel que le mélange de monomère A et de monomère B comprend des groupes polymérisables, dont au moins 90 % sont présents dans des monomères ayant au moins deux groupes polymérisables, en une concentration allant de 3,2 à 15 mmoles/g.

En particulier, le polymère à cristaux liquide comprend essentiellement ou consiste en un mélange des monomères A et B définis précédemment.

Le polymère à cristaux liquide présente notamment un pas d'hélice supérieur à 450 nm, notamment allant de 455 nm à 5000 nm, en particulier allant de 455 nm à 1000 nm, voire allant de 455 nm à 650 nm.

Le monomère A peut posséder un poids moléculaire moyen en poids allant de 150 à 800, et notamment allant de 460 à 625. Le monomère A peut être en particulier un dérivé de dibenzoate d'hydroquinone non substitué.

Le monomère B peut avoir un poids moléculaire moyen en poids allant de 500 à 1000, et notamment allant de 500 à 700.

Le polymère à cristaux liquides peut posséder un poids moléculaire moyen en poids inférieur à 625.

Le polymère à cristaux liquides défini précédemment peut être préparé suivant les procédés connus dans l'état de la technique, tels que ceux décrits dans les documents US 5362315, US 5807497, à partir du mélange de monomère décrit précédemment.

La polymérisation du mélange de monomères orienté peut, de manière déjà connue, se faire par exemple de façon radicalaire avec utilisation d'initiateurs thermiques du commerce, en utilisant des rayons d'électrons ou de la lumière UV en combinaison avec des photoinitiateurs du commerce, ou bien par des réactions d'addition ou de condensation.

La réticulation des mélanges de monomères, dans l'état structurel chiral, se fait de préférence au moyen d'une polyréaction qui, selon le type des groupes polymérisables, polycondensables ou polyadditionnables, se déroule sous la forme d'une polymérisation radicalaire, ionique ou catalysée au métal, ou d'une réaction de polycondensation ou d'une réaction de polyaddition.

L'initiation de la polymérisation radicalaire peut s'effectuer au moyen d'initiateurs correspondants ou par un rayonnement par UV, en utilisant des photoinitiateurs du commerce ou par un rayonnement à haute énergie, tel qu'un rayonnement d'électrons. Un avantage de la polymérisation thermique des radicaux ou de la polymérisation via un durcissement aux rayons d'électrons réside dans le fait qu'au mélange polymérisable peut également être ajouté un agent de protection contre la lumière, tel qu'un absorbeur d'UV (UVA) ou des capteurs de radicaux (HALS), pour stabiliser les pigments ou les films résultants face à la lumière UV, par exemple pour des applications extérieures, sans entraîner des pertes au niveau de la conversion de polymérisation, tel que ceci est le cas lors du durcissement aux UV, du fait de l'effet d'écrantage du photoinitiateur par un UVA. Il n'y a ainsi aucune diminution de la densité de réticulation.

Si le durcissement des films se fait de façon peroxydique ou par un rayonnement d'électrons, le mélange de monomères contient de préférence des agents de protection contre la lumière, du commerce, tels que des absorbeurs à UV ou des capteurs de radicaux, en une concentration globale de 0,5 à 5 % en poids.

Outre les photostabilisateurs, les mélanges de monomères peuvent également contenir d'autres additifs usuels visant à inhiber l'oxydation, à inhiber la polymérisation, ou des additifs visant à améliorer les propriétés rhéologiques. De plus, les charges absorbantes, telles que les pigments ou la suie, ainsi que des colorants ou des pigments à fluorescence peuvent être contenus.

Le film obtenu après la polymérisation est ensuite broyé en particules, notamment sous forme de plaquettes.

De préférence, les particules de polymère à cristaux liquides ont une plus grande taille allant de 1 µm à 3 mm, et de préférence allant de 30 µm à 500 µm. Ces particules sont avantageusement en forme de plaquettes.

Les particules peuvent être séparées (triées) par un procédé à sélectivité de la taille de grains.

De tels polymères et leurs particules sont décrits dans la demande EP-A-1046692.

Comme particules de polymère à cristaux liquides, on peut notamment utiliser celles connues sous le nom CTFA Polyacrylate-4 et vendus sous les dénominations « HELICONE® HC Sapphire », « HELICONE® HC Scarabeus », « HELICONE® HC Jade », « HELICONE® HC Maple », « HELICONE® HC XL Sapphire », « HELICONE® HC XL Scarabeus », « HELICONE® HC XL Jade », « HELICONE® HC XL Maple » par la société WACKER.

La matière colorante présente dans la composition peut être également choisie parmi les agents de coloration photochromes.

Un agent de coloration photochrome est un agent ayant la propriété de changer de couleur lorsqu'il est éclairé par de la lumière ultraviolette et de reprendre sa couleur initiale lorsqu'il n'est plus éclairé par cette lumière ou encore de passer d'un état non coloré à un état coloré et inversement. En particulier, cet agent présente des couleurs différentes selon qu'il est éclairé par de la lumière naturelle ou de la lumière artificielle.
Les agents photochromes utilisables dans l'invention sont en particulier ceux décrits dans les documents JP-A-09/165532, EP-A-709728, JP-A-07/258580, JP-A-07/223816, EP-A-624553, JP-A-08/337422, JP-A-07/025617, EP-A-359909.

Plus précisément, les agents coloration photochromes utilisables dans l'invention sont les spiro-oxazines et leurs dérivés comme les spiroindolino-naphto-oxazines, les spironaphtoxazines, le naphtopyrane et ses dérivés, les spiropyrannes, comme les indolinospirobenzopyrannes, les nitrobenzylpyridines, les spirolanes, les oxydes de titane ou de zinc dopé par du fer. A titre d'exemple, on peut citer les agents photochromes du type dérivés de naphtopyrane vendus par la société PPG sous les références Photosol 5-68 Photochromic Dye, Photosol 7-49 Photochromic Dye, Photosol 7-106 Photochromic Dye, Photosol 0265 Photochromic Dye, Photosol 0272 Photochromic Dye, ces agents présentant deux couleurs différentes selon qu'ils sont excités ou non par des U.V. On peut aussi utiliser des aluminosilicates dopés notamment par les groupements S, Se, SO₄²⁻, WO₄²⁻, ou OH ou encore par des ions métalliques notamment de Fe, Cr, Mn, Co, Ni, tels que ceux décrits dans la demande EP-A-847751.

La matière colorante présente dans la composition peut être également choisie parmi les agents (ou substance) fluorescents.

Les agents fluorescents sont bien connus de l'homme du métier. Ilss peuvent être des pigments ou des colorants. Par pigments, on entend des particules, minérales ou organiques, insolubles dans la composition. Par colorant, on entend des composés chimiques solubilisés dans la composition. Les colorants peuvent être hydrosolubles ou liposolubles. Les substances fluorescentes sont par exemple décrites dans « Luminescent materials (fluorescent daylight), Encyclopedia of Chemical Technology, Kirk-Othmer", vol 14, p. 546-569, 3ème édition, 1981, Wiley.

Au sens de la présente invention, on entend par agent fluorescent une substance qui, sous l'effet de rayons ultraviolet et/ou de la lumière visible, réémet dans le visible la portion de lumière qu'elle a absorbé sous la même couleur que celle qu'elle reflète naturellement. La couleur réfléchie naturellement est ainsi renforcée par la couleur réémise et apparaît extrêmement brillante.

Comme agent fluorescent, on peut utiliser des substances fluorescentes inorganiques comme celles décrites dans la demande JP05-117127 et en particulier les substances fluorescentes inorganiques à base d'oxyde de zinc.

Comme agents fluorescent , on peut également utiliser des substances fluorescentes organiques comme les pigments fluorescents à la lumière du jour : ces pigments sont généralement fabriqués à partir de colorants fluorescents qui sont préalablement dissous dans une résine support afin d'obtenir une solution solide laquelle est ensuite broyée en une poudre de particules de résine présentant des propriétés fluorescentes. La préparation de tels pigments fluorescents est écrite dans EP 0 370 470, US 2, 851, 424, US 3, 711, 604, US 3, 856, 550 et US 2, 938, 878.

Les pigments fluorescents convenant particulièrement bien à la présente invention peuvent ainsi être choisis parmi les résines colorées de polyamide et/ou de formaldéhyde/benzoguanamine et/ou de melamine/formaldéhyde/sulfonamide, parmi les co-condensats aminotriazine/formaldéhyde/sulfonamide colorés et/ou parmi les paillettes polyester métallisées et/ou leurs mélanges. Ces pigments fluorescents peuvent aussi se présenter sous la forme de dispersions aqueuses de pigments fluorescents.

Comme pigments fluorescents convenant particulièrement bien à la présente invention, on peut citer le co-condensat aminotriazine/formaldehyde/sulfonamide fluorescent coloré en rose de taille moyenne des particules 3-4 microns vendu sous la dénomination commerciale « Fiesta Astral Pink FEX-1 » et le co-condensat aminotriazine/formaldehyde/sulfonamide fluorescent coloré en bleu de taille moyenne des particules 3-4,5 microns vendu sous la dénomination commerciale « Fiesta Comet Blue FTX-60 » par la société Swada ou encore la résine benzoguanamine/formaldéhyde recouverte de résine formaldéhyde/urée et colorée en jaune vendue sous la dénomination commerciale « FB-205 Yellow » et la résine benzoguanamine/formaldéhyde recouverte de résine formaldéhyde/urée et colorée en rouge vendue sous la dénomination commerciale « FB-400 Orange Red » par la société UK SEUNG CHEMICAL, la résine polyamide colorée en orange vendue sous la dénomination commerciale « Flare 911 Orange 4 » par la société Sterling Industrial Colors.

Les agents fluorescents peuvent être aussi choisi parmi les azurants optiques, qui sont des substances fluorescentes organiques blanches.

Les azurants optiques sont des composés bien connus de l'homme du métier. De tels composés sont décrits dans "Fluorescent Whitening Agent, Encyclopedia of Chemical Technology, Kirk-Othmer", vol 11, p. 227-241, 4ème édition, 1994, Wiley.

Leur utilisation en cosmétique à titre d'agents de blanchiment de la peau par voie optique, tire en particulier profit du fait qu'ils sont constitués de composés chimiques dotés de propriétés de fluorescence qui absorbent dans l'ultraviolet (absorption maximale à une longueur d'onde inférieure à 400 nm) et réémettent de l'énergie par fluorescence pour une longueur d'onde comprise entre 380 et 830 nm.

On peut plus particulièrement les définir comme des composés qui absorbent essentiellement dans l'UVA entre 300 et 390 nm et réémettent essentiellement entre 400 et 525 nm.

Leur effet éclaircissant repose plus particulièrement sur une émission d'énergie comprise entre 400 et 480 nm, ce qui correspond à une émission dans le bleu du domaine visible, qui contribue, quand cette émission a lieu sur la peau, à l'éclaircir visuellement.

Sont notamment connus à titre d'azurants optiques les dérivés du stilbène, en particulier les polystyrylstilbènes et les triazinstilbènes, les dérivés coumariniques, en particulier les hydroxycoumarines et les aminocoumarines, les dérivés oxazole, benzoxazole, imidazole, triazole, pyrazoline, les dérivés du pyrène, les dérivés de porphyrine et leurs mélanges.

De tels composés sont largement commercialisés. Il s'agit notamment des dérivés suivants :
- le dérivé stilbénique de naphto-triazole vendu sous la dénomination commerciale « Tinopal GS », le di-styryl-4,4 biphényle sulfonate di-sodique (nom CTFA : disodium distyrylbiphenyl disulfonate) vendu sous la dénomination commerciale « Tinopal CBS-X », le dérivé cationique d'aminocoumarine vendu sous la dénomination commerciale « Tinopal SWN CONC. », le 4,4'-bis[(4,6-dianilino-1,3,5-triazin-2-yl)amino]stilbène-2,2'-disulfonate de sodium vendu sous la dénomination commerciale « Tinopal SOP », l'acide 4,4-bis-[(4-anilino-6-bis(2-hydroxyéthyl)amino-1,3,5-triazin-2-yl)amino]stilbène-2,2 -disulfonique vendu sous la dénomination commerciale «Tinopal UNPA-GX », le 4,4-bis-[anilino-6-morpholine-1,3,5-triazin-2-yl)amino]stilbène vendu sous la dénomination commerciale « Tinopal AMS-GX », le 4,4 -bis-[(4-anilino-6-(2-hydroxyéthyl)méthyl amino-1,3,5-triazin-2-yl)amino]stilbène-2,2 - disodium sulfonate vendu sous la dénomination commerciale « Tinopal 5BM-GX », tous par la société CIBA Spécialités Chimiques ;
- le 2,5 thiophène di-yl bis(5 ter-butyl-1,3 benzoxazole) vendu sous la dénomination commerciale « Uvitex OB » par la société CIBA ;
- le dérivé anionique du di-aminostilbène en dispersion dans l'eau vendu sous la dénomination commerciale « Leucophor BSB liquide » par la société CLARIANT ; et
- leurs mélanges.

Les azurants optiques utilisables dans la présente invention peuvent aussi se présenter sous la forme de copolymères, par exemple d'acrylates et/ou de méthacrylates, greffés par des groupements azurants optiques comme décrits dans la demande FR 99 10942.

Conviennent tout particulièrement à l'invention le di-styryl-4,4 biphényle sulfonate di-sodique, le 4,4'-bis[(4,6-dianilino-1 ,3,5-triazin-2-yl)amino]stilbène-2,2'-disulfonate de sodium, le 2,5 thiophène di-yl bis(5 ter-butyl-1,3 benzoxazole) et leurs mélanges.

La matière colorante peut être présente dans la composition selon l'invention en une teneur allant de 1 % à 80 % en poids, par rapport au poids total de la composition, de préférence allant de 1 % à 70 % en poids, de préférence allant de 1 % à 60 % en poids, préférentiellement allant de 5 % à 50 % en poids, plus préférentiellement allant de 5 % à 40 % en poids, et encore plus préférentiellement allant de 5 % à 30 % en poids.

La composition selon l'invention peut contenir une matière colorante additionnelle pulvérulente différente des matières colorantes décrites précédemment.
La matière colorante additionnelle pulvérulente peut être choisie parmi les pigments et les nacres, différents des matières colorantes décrites précédemment.

Par pigments, il faut comprendre des particules de toute forme, blanches ou colorées, minérales ou organiques, insolubles dans le milieu physiologique, destinées à colorer la composition.
Par nacres, il faut comprendre des particules de toute forme irisées, notamment produites par certains mollusques dans leur coquille ou bien synthétisées.

Les pigments peuvent être présents dans la composition, en un teneur allant de 0.5 % à 30 % en poids, par rapport au poids de la composition, de préférence allant de 0,5 % à 20 % en poids.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique.

Parmi les pigments organiques, on peut citer le noir de carbone, les pigments de type D & C, et les laques à base de carmin de cochenille, de baryum, strontium, calcium, aluminium.

Les nacres peuvent être présentes dans la composition en une teneur allant de 0,5 % à 50 % en poids, par rapport au poids total de la composition, de préférence allant de 0,5 % à 30 % en poids.

Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth.

La composition selon l'invention peut comprendre des charges additionnelles différentes des particules solides ayant un indice de réfraction allant de 1,2 à 1,45 décrites précédemment.

Par charges, il faut comprendre des particules de toute forme, incolores ou blanches, minérales ou de synthèse, insolubles dans le milieu de la composition quelle que soit la température à laquelle la composition est fabriquée.

Les charges peuvent être minérales ou organiques de toute forme, plaquettaires, sphériques ou oblongues, quelle que soit la forme cristallographique ( par exemple feuillet, cubique, hexagonale, orthorombique, etc) . On peut citer le talc, le mica, la séricite, les particules de silice ayant indice de réfraction supérieur à 1,45, le kaolin, les poudres de polyamide (Nylon®), les poudres de poly-β-alanine, les poudres de polyéthylène, la lauroyl-lysine, l'amidon, le nitrure de bore, les microsphères creuses polymériques telles que celles de chlorure de polyvinylidène/acrylonitrile comme l'Expancel® (Nobel Industrie), les poudres de copolymères d'acide acrylique, les particules de polyméthacrylate de méthyle, les poudres de copolymère diméthacrylate d'éthylèneglycol et de méthacrylate de lauryle vendue sous la dénomination POLYTRAP® 6603 adsorber par la société RP Scherer, le carbonate de calcium précipité, le carbonate et l'hydro-carbonate de magnésium, l'hydroxyapatite, les microcapsules de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium ; et leurs mélanges.

Les charges additionnelles sont de préférence choisies parmi le mica, le nitrure de bore, les particules de silice ayant indice de réfraction supérieur à 1,45, les poudres de polyamide (Nylon@), les poudres de polyéthylène, les poudres de copolymère diméthacrylate d'éthylèneglycol et de méthacrylate de lauryle, les particules de polyméthacrylate de méthyle, et leurs mélanges.

Les charges additionnelles peuvent être présentes dans la composition en une teneur telle que l'effet optique des matières colorantes particulières ne soit pas altéré. Notamment, les charges peuvent être présentes en une teneur allant de 0,5 % à 50 % en poids, par rapport au poids total de la composition, de préférence allant de 0,5 % à 30 % en poids, et préférentiellement allant de 0,5 % à 20 % en poids.

Avantageusement, la composition selon l'invention peut comprendre une teneur totale en poudres additionnelles distinctes des particules solides ayant un indice de réfraction allant de 1,2 à 1,45 (donc matière colorante + charges) allant de 20 % à 80 % en poids, par rapport au poids total de la composition, de préférence allant de 30 % à 70 % en poids, préférentiellement allant de 35 % à 65 % en poids, et plus préférentiellement allant de 40 % à 60 % en poids.

De préférence, les particules solides d'organopolysiloxane réticulé élastomère ayant un indice de réfraction allant de 1,2 à 1,45 et les autres particules additionnelles distinctes des particules solides d'organopolysiloxane réticulé élastomère ayant un indice de réfraction allant de 1,2 à 1,45 (donc matière colorante + charges) sont présentes dans la composition selon l'invention en des teneurs telles que le rapport pondéral particules d'organopolysiloxane réticulé/particules additionnelles va de 0,4/1 à 2,5/1, de préférence va de 0,4/1 à 2/1, préférentiellement va de 0,4/1 à 1,5/1, plus préférentiellement va de 0,4/1 à 1,3/1, et encore plus préférentiellement va de 0,6/1 à 1,3/1.

La composition peut contenir d'autres ingrédients cosmétiques usuels pouvant être choisis notamment parmi les antioxydants, les parfums, les conservateurs, les neutralisants, les fibres, les tensioactifs, les cires, les filtres solaires, les vitamines, les hydratants, les composés auto-bronzants, les actifs antirides.

Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

La composition de maquillage est notamment une composition non rincée.

En particulier, la composition selon l'invention peut être une composition poudreuse. On entend par composition poudreuse une composition comprenant une phase pulvérulente et ayant une texture pouvant être sous forme de poudre libre, de composition solide (poudre compacte ou poudre pressée), ou sous forme de composition pâteuse. La composition poudreuse n'est pas une composition liquide (au sens d'une composition qui s'écoule sous son propre poids à température ambiante (25 °C) ). Avantageusement, la composition poudreuse selon l'invention est une composition solide.

On entend par composition solide une composition qui ne s'écoule pas sous son propre poids à température ambiante (25 °C) au bout d'une heure.

Aussi, selon un mode de réalisation particulier de l'invention, l'invention a également pour objet une composition de maquillage poudreuse comprenant des particules solides d'organopolysiloxane réticulé élastomère, de l'eau et une matière colorante choisie parmi les particules réfléchissantes différentes des particules réfléchissantes comprenant un substrat de mica, les agents de coloration goniochromatiques, les agents de coloration photochromes, les agents fluorescents, et leurs mélanges.

L'invention est illustrée plus en détails par les exemples décrits ci-après.

### Exemple 1 :

On a préparé un fard à paupières ayant la composition suivante :
- Particules de polydiméthylsiloxane réticulé élastomère en dispersion aqueuse à 63 % de polymère réticulé (BY 29-119 de Dow Corning) 45 g soit 28,35 g MA
- Paillettes de verre enrobées d'argent (Metashine ME 2040 PS de Nippon Sheet Glass) 20 g
- Mica oxyde de titane (Flamenco Blue de Engelhard) 26,6 g
- Mica-oxyde de titane-bleu ferrique (Colorona Dark Blue de Merck) 2,4 g
- Glycérine 5 g
- Conservateur 1 g

Après mélange des ingrédients, la composition est soit tamisée pour obtenir une poudre libre soit est conditionnée dans une coupelle par pressage pour obtenir un produit compact. Le produit après application sur la peau confère un maquillage présentant un aspect métallique argenté éclatant.

### Exemple 2 :

On a préparé un fard à joue ayant la composition suivante :
- Particules de polydiméthylsiloxane réticulé élastomère en dispersion aqueuse à 63 % de polymère réticulé
   (BY 29-119 de Dow Corning) 45 g soit 28,35 g MA
- Pigment à structure multicouche interférentiel
   (Sicopearl fantastico rose de BASF) 20 g
- Mica-oxyde de fer brun-oxyde de titane
   (Flamenco Satin Red 460M de Engelhard) 29 g
- Glycérine 5 g
- Conservateur 1 g

Après mélange des ingrédients, la composition est soit tamisée pour obtenir une poudre libre soit est conditionnée dans une coupelle par pressage pour obtenir un produit compact. Le produit après application sur la peau confère un maquillage présentant des reflets colorés intenses. Selon l'angle d'observation du maquillage, on perçoit nettement un changement de couleur allant du rose au doré.

### Exemple 3 :

Cn a préparé un fond de teint ayant la composition suivante :
- Particules de polydiméthylsiloxane réticulé élastomère en dispersion
   aqueuse à 63 % de polymère réticulé
   (BY 29-119 de Dow Corning) 50 g soit 31,5 g MA
- Matière colorante photochrome
   (Photolight Pearl Red-80 de chez Nihon Kokken) 10g
- Pigments (oxyde de fer, dioxyde de titane) 5g
- Poudre de nylon (Orgasol^{®} 2002 extra D NAT COS d'ATOFINA) 20g
- Glycérine 5g
- Propylène glycol 5g
- Poudre de copolymère diméthacrylate d'éthylèneglycol et de méthacrylate de lauryle vendue sous la dénomination POLYTRAP^{®} 6603 adsorber par la société RP Scherer 4 g
- Conservateur 1 g

Après mélange des ingrédients, la composition est soit tamisée pour obtenir une poudre libre soit est conditionnée dans une coupelle par pressage pour obtenir un produit compact. Le produit après application sur la peau confère un maquillage dont la teinte change sous l'effet d'une excitation UV (par exemple sous une lampe UV).

### Exemple 4 :

On a préparé un fard à paupières ayant la composition suivante :
- Particules de polydiméthylsiloxane réticulé élastomère en dispersion
   aqueuse à 63 % de polymère réticulé
   (BY 29-119 de Dow Corning) 40 g soit 25,2 g MA
- Glycérine 5g
- Butylène glycol 5g
- Pigments goniochromatiques cristaux liquides (Helicone^{®} HC Scarabeus -de la société Wacker) 20 g
- Mica oxyde de titane (Flamenco Blue de Engelhard) 15 g
- Pigments (oxyde de fer noir) 5g
- Poudre de nylon (Orgasol^{®} 2002 extra D NAT COS d'ATOFINA) 9 g
- Conservateur 1 g

Après mélange des ingrédients, la composition est soit tamisée pour obtenir une poudre libre soit est conditionnée dans une coupelle par pressage pour obtenir un produit compact. Le produit après application sur la peau confère un maquillage présentant des reflets colorés intenses.

### Exemple 5 :

On a préparé une poudre de maquillage ayant la composition suivant :
- Particules de polydiméthylsiloxane réticulé élastomère en dispersion aqueuse à 63 % de polymère réticulé
   (BY 29-119 de Dow Corning) 45 g soit 28,35 g MA
- Glycérine 5 g
- Cyclopentadiméthylsiloxane 5 g
- Plaquettes de borosilicate de calcium et de sodium enrobée de
   dioxyde de titane et de dioxyde d'étain (92,5/7/0,5) (REFLECKS VISIONS OF VIOLET G 530L - Engelhard) 24 g
- Mica oxyde de titane (Flamenco Blue de Engelhard) 15 g
- Pigments (oxyde de fer noir) 5 g
- Conservateur 1 g

Après mélange des ingrédients, la composition est soit tamisée pour obtenir une poudre libre soit est conditionnée dans une coupelle par pressage pour obtenir un produit compact. Le produit après application sur la peau confère un maquillage présentant des reflets colorés intenses.

## Revendications

1. Composition de maquillage poudreuse comprenant des particules solides d'organopolysiloxane réticulé élastomère ayant un indice de réfraction allant de 1,2 à 1,45, lesdites particules se présentant sous la forme d'une dispersion aqueuse, un liant liquide ayant un indice de réfraction allant de 1,2 à 1,6, et une matière colorante choisie parmi les particules réfléchissantes différentes des particules réfléchissantes comprenant un substrat de mica, les agents de coloration goniochromatiques, les agents de coloration photochromes, les agents fluorescents, et leurs mélanges.

2. Composition selon la revendication 1, **caractérisée par le fait que** l'indice de réfraction des dites particules solides va de 1,25 à 1,45, de préférence va de 1,3 à 1,45, préférentiellement va de 1,35 à 1,45, plus préférentiellement va de 1,38 à 1,45, et encore plus préférentiellement va de 1,40 à 1,45.

3. Composition selon l'une quelconque des revendications précédentes, **caractérisée par le fait que** l'organopolysiloxane réticulé est choisi parmi ceux obtenus :
- par réaction d'addition réticulation de diorganosiloxane contenant au moins un hydrogène lié au silicium et de diorganopolysiloxane ayant des groupements à insaturation éthylénique liés au silicium ;
- par réaction de condensation réticulation déhydrogénation entre un diorganopolysiloxane à terminaisons hydroxyle et un diorganopolysiloxane contenant au moins un hydrogène lié au silicium ;
- par réaction de condensation réticulation d'un diorganopolysiloxane à terminaisons hydroxyle et d'un organopolysilane hydrolysable ;
- par réticulation thermique d'organopolysiloxane ;
- par réticulation d'organopolysiloxane par radiations de haute énergie.

4. Composition selon l'une quelconque des revendications précédentes, **caractérisée par le fait que** l'organopolysiloxane réticulé est obtenu par réaction d'addition réticulation (A) de diorganopolysiloxane contenant au moins deux hydrogènes liés chacun à un silicium, et (B) de diorganopolysiloxane ayant au moins deux groupements à insaturation éthylénique liés au silicium, notamment en présence (C) de catalyseur platine.

5. Composition selon l'une quelconque des revendications précédentes, **caractérisée par le fait que** l'organopolysiloxane réticulé est obtenu par réaction de diméthylpolysiloxane à terminaisons diméthylvinylsiloxy et de méthylhydrogénopolysiloxane à terminaisons triméthylsiloxy, en présence de catalyseur platine.

6. Composition selon l'une quelconque des revendications précédentes, **caractérisée par le fait que** l'organopolysiloxane réticulé est un élastomère non émulsionnant.

7. Composition selon l'une quelconque des revendications précédentes, **caractérisée par le fait que** les dites particules solides d'organopolysiloxane réticulé élastomère ayant un indice de réfraction allant de 1,2 à 1,45 sont présentes en une teneur allant de 5 % à 80 % en poids, par rapport au poids total de la composition, de préférence allant de 10 % à 70 % en poids, de préférence allant de 10 % à 60 % en poids, préférentiellement allant de 10 % à 50 % en poids, plus préférentiellement allant de 15 % à 50 % en poids, plus préférentiellement allant de 20 % à 45 % en poids, et encore plus préférentiellement allant de 20 % à 40 % en poids.

8. Composition selon l'une quelconque des revendications précédentes, **caractérisée par le fait que** le liant liquide a un indice de réfraction allant de 1,2 à 1,5, de préférence allant de 1,25 à 1,45, et préférentiellement allant de 1,3 à 1,45.

9. Composition selon l'une quelconque des revendications précédentes, **caractérisée par le fait que** le liant liquide est choisi parmi l'eau, les solvants organiques miscibles à l'eau, les huiles, et leurs mélanges.

10. Composition selon l'une quelconque des revendications précédentes, **caractérisée par le fait que** le liant liquide est choisi parmi l'eau et les solvants organiques miscibles à l'eau, et leurs mélanges.

11. Composition selon la revendication 9 ou 10, **caractérisée par le fait que** les solvants organiques miscibles à l'eau sont choisis parmi les monoalcools ayant de 2 à 6 atomes de carbone ; les polyols ayant de 2 à 20 atomes de carbones, de préférence ayant de 2 à 10 atomes de carbone, et préférentiellement ayant de 2 à 6 atomes de carbones ; les éthers de glycol ayant de 3 à 16 atomes de carbone ; et leurs mélanges.

12. Composition selon l'une quelconque des revendications 9 à 12, **caractérisée par le fait que** les solvants organiques miscibles à l'eau sont choisis parmi l'éthanol, l'isopropanol, la glycérine, le propylène glycol, le butylène glycol, le pentylène glycol, l'hexylène glycol, le dipropylène glycol, le diéthylène glycol, les alkyl(C₁-C₄)éther de mono, di- ou tripropylène glycol, les alkyl(C₁-C₄)éthers de mono, di- ou triéthylène glycol, et leurs mélanges.

13. Composition selon la revendication 9, **caractérisée par le fait que** les huiles sont choisies parmi les huiles hydrocarbonées, les huiles siliconées, les huiles fluorées, et leurs mélanges.

14. Composition selon la revendication 9 ou 10, **caractérisée par le fait que** les huiles sont choisies parmi les huiles volatiles.

15. Composition selon l'une quelconque des revendications précédentes, **caractérisée par le fait que** le liant liquide est présent en une teneur allant de 5 % à 60 % en poids, par rapport au poids total de la composition, de préférence allant de 5 % à 50 % en poids, de préférence allant de 5 % à 45 % en poids, préférentiellement allant de 10 % à 40 % en poids, et plus préférentiellement allant de 15 % à 35 % en poids.

16. Composition selon l'une quelconque des revendications précédentes, **caractérisée par le fait que** la matière colorante est choisie parmi les particules réfléchissantes différentes des particules réfléchissantes comprenant un substrat de mica.

17. Composition selon l'une quelconque des revendications précédentes, **caractérisée par le fait que** les particules réfléchissantes différentes des particules réfléchissantes comprenant un substrat de mica sont choisies parmi :
- les particules à substrat naturel ou synthétique enrobé au moins partiellement par au moins une couche d'au moins un métal,
- les particules à substrat synthétique, différent du mica, enrobé au moins partiellement par au moins une couche d'au moins un composé métallique et notamment d'un oxyde métallique,
- les particules formées d'un empilement d'au moins deux couches de matériaux à indices de réfraction différents, l'une au moins de ces couches pouvant être un polymère, et
- les particules métalliques ;
- et leurs mélanges.

18. Composition selon l'une quelconque des revendications précédentes, **caractérisée par le fait que** les particules réfléchissantes sont choisies parmi les particules à substrat enrobé au moins partiellement par au moins une couche d'au moins un métal.

19. Composition selon la revendication précédente, **caractérisée par le fait que** le métal est choisi parmi Ag, Au, Cu, Al, Zn, Ni, Mo, Cr et leurs alliages.

20. Composition selon la revendication précédente, **caractérisée par le fait que** les particules réfléchissantes sont choisies parmi les particules à substrat synthétique, différent du mica, enrobé au moins partiellement par au moins une couche d'au moins un composé métallique et notamment d'un oxyde métallique.

21. Composition selon la revendication précédente, **caractérisée par le fait que** le composé métallique est choisi parmi les oxydes de titane, notamment TiO₂, de fer, d'étain, notamment Fe₂O₃, de chrome, le sulfate de baryum et les composés MgF₂, CeF₃, ZnS, ZnSe, SiO₂, Al₂O₃, MgO, Y₂O₃, SeO₃, SiO, HfO₂, ZrO₂, CeO₂, Nb₂O₅, Ta₂O₅ et MoS₂ et leurs mélanges.

22. Composition selon la revendication précédente, **caractérisée par le fait que** le composé métallique est un oxyde de titane, un oxyde de fer ou un oxyde d'étain ou un de leurs mélanges.

23. Composition selon l'une quelconque des revendications 20 à 22, **caractérisée par le fait que** le substrat synthétique est choisi parmi les substrats monomatière, multi-matériaux, les substrats organiques, les substrats inorganiques, les verres, les céramiques, les oxydes métalliques, les alumines, les silices, les silicates, notamment les aluminosilicates et les borosilicates.

24. Composition selon la revendication précédente, **caractérisée par le fait que** le substrat synthétique est choisi parmi les verres.

25. Composition selon la revendication 16, **caractérisée par le fait que** les particules réfléchissantes sont choisies parmi les particules formées d'un empilement d'au moins deux couches de polymères à indices de réfraction différents.

26. Composition selon l'une quelconque des revendications précédentes, **caractérisée par le fait que** les particules réfléchissantes sont choisies parmi par des particules métalliques réfléchissantes.

27. Composition selon la revendication précédente, **caractérisée par le fait que** les particules métalliques réfléchissantes sont choisies parmi les particules de d'or, .d'argent, d'aluminium, de cuivre.

28. Composition selon l'une quelconque des revendications précédentes, **caractérisée par le fait qu'**elle comprend des agents de coloration goniochromatiques.

29. Composition selon la revendication précédente, **caractérisée par le fait que** l'agent de coloration goniochromatique est à structure multicouches interférentielles.

30. Composition selon la revendication précédente, **caractérisée par le fait que** les agents de coloration goniochromatiques sont des pigments à structure multicouche interférentielle comportant au moins deux couches, chaque couche étant réalisée en au moins un matériau choisi dans le groupe constitué par les matériaux suivants : MgF₂, CeF₃, ZnS, ZnSe, Si, SiO₂, Ge, Te, Fe₂O₃, Pt, Va, Al₂O₃, MgO, Y₂O₃, S₂O₃, SiO, HfO₂, ZrO₂, CeO₂, Nb₂O₅, Ta₂O₅, TiO₂, Ag, Al, Au, Cr, Cu, Rb, Ti, Ta, W, Zn, MoS₂, cryolithe, alliages, polymères et leurs associations.

31. Composition selon l'une des revendications 29 ou 30, telle que les agants de coloration goniochromatiques sont des pigments à structure multicouche interférentielle choisie dans le groupe constitué par les structures : Al/SiO₂/Al/SiO₂/Al ; Cr/MgF₂/Al/MgF₂/Cr ; MoS₂/SiO₂/Al/SiO₂/MoS₂ ; Fe₂O₃/SiO₂/Al/SiO₂/Fe₂O₃ ; Fe₂O₃/SiO₂/Fe₂O₃/SiO₂/Fe₂O₃; MoS₂/SiO₂/mica-oxyde/SiO₂/MoS₂ ; Fe₂O₃/SiO₂/mica-oxyde/SiO₂/Fe₂O₃ et leurs associations.

32. Composition selon la revendication 28, **caractérisée par le fait que** l'agent de coloration goniochromatique est un agent de coloration à cristaux liquides.

33. Composition selon la revendication précédente, **caractérisée par le fait que** l'agent de coloration goniochromatique à cristaux liquides est obtenu par polymérisation d'un mélange de monomères comprenant :
- a) au moins un premier monomère A de formule (I) Y1-A1-M1-A2-Y2 dans laquelle
- i) Y1 et Y2 , identiques ou différents, représentent un groupe acrylate ou méthacrylate, de préférence un groupe acrylate ;
- ii) A1 et A2, identiques ou différents, représentent un groupement de formule
- CnH₂n-, dans laquelle n est un nombre entier allant de 1 à 20, notamment allant de 2 à 6, et en particulier égal à 4 ;
- iii) M1 désigne un groupement de formule générale (I') -R₁-X₁-R₂-X₂-R₃-X₃-R₄-, dans laquelle R₁ et R₄ désignent -O-, R₂ et R₃ désignent -COO-,
et X₁,X₂,X₃ sont un groupement 1,4 phénylène, le groupe carbonyle - CO- respectivement de R₂ et de R₃ étant lié respectivement au groupement X₁, X₃
et
- b) au moins un deuxième monomère B chiral de formule (II) V1-W1-Z-W2-V2, dans laquelle
- i) V1 désigne un groupement acrylate ou méthacrylate, et notamment un groupe acrylate, et V2 désigne un groupement alkyle en C₁-C₂₀, un alkoxy en C₁-C₂₀, un alkoxy(C₁-C₂₀)carbonyle, -OH, et de préférence désigne un groupement alkoxy en C₁-C₂₀, notamment en C₁-C₄, et en particulier un groupement méthoxy ;
ii) W1 représente un groupement divalent de formule -X'1-CO-O-,
W2 représente un groupement divalent de formule -O-CO-X'1-,
dans lesquelles X'1 désigne un groupe 1,4-phénylène,
et Z désigne un groupement chiral à deux liaisons, issu du groupement dianhydrohexite, en particulier un radical divalent de formule :

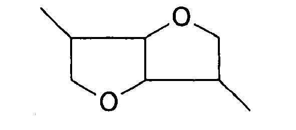

34. Composition selon la revendication précédente, **caractérisée par le fait que** l'agent de coloration à cristaux liquides est obtenu à partir d'un mélange de monomères comprenant de 70 à 99 % en poids de monomère A et de 1 à 30 % en poids de monomère B, par rapport au poids total de monomère A et de monomère B, et notamment comprenant de 90 à 95 % en poids de monomère A et de 5 à 10 % en poids de monomère B.

35. Composition selon l'une quelconque des revendications précédentes, **caractérisée par le fait qu'**elle comprend un agent de coloration photochrome.

36. Composition selon l'une quelconque des revendications précédentes, **caractérisée par le fait que** l'agent de coloration photochrome est choisi parmi les spiro-oxazines et leurs dérivés, les spironaphtoxazines, le naphtopyrane et ses dérivés, les spiropyrannes, les nitrobenzylpyridines, les spirolanes, les oxydes de titane ou de zinc dopé par du fer, les aluminosilicates dopés par des ions métalliques ou par un groupement choisi parmi Se, S, OH, SO₄²⁻ WO₄²⁻.

37. Composition selon l'une quelconque des revendications précédentes, **caractérisée par le fait qu'**elle comprend un agent fluorescent.

38. Composition selon la revendication précédente, **caractérisée par le fait que** l'agent fluorescent est choisi parmi les pigments fluorescents organiques.

39. Composition selon la revendication précédente, **caractérisée par le fait que** lesdits pigments fluorescents sont choisis parmi les résines colorées de polyamide et/ou de formaldéhyde/benzoguanamine et/ou de melamine/formaldéhyde/sulfonamide, parmi les co-condensats aminotriazine/formaldéhyde/sulfonamide colorés et/ou parmi les paillettes polyester métallisées et/ou leurs mélanges.

40. Composition selon la revendication 37, **caractérisée par le fait que** l'agent fluorescent est choisi parmi les azurants optiques.

41. Composition cosmétique selon la revendication précédente, **caractérisée par le fait que** l'azurant optique est choisi parmi les dérivés du stilbène, les dérivés coumariniques, les dérivés oxazole, benzoxazole, imidazole, triazole, pyrazolines, les dérivés du pyrène, les dérivés de porphyrine et leurs mélanges.

42. Composition selon la revendication 40 ou 41 **caractérisée par le fait que** l'azurant optique est choisi parmi :
- le dérivé stilbénique de naphto-triazole, le di-styryl-4,4' biphényle sulfonate disodique (nom CTFA : disodium distyrylbiphenyl disulfonate), le 4,4'-bis[(4,6-dianilino-1,3,5-triazin-2-yl)amino]stilbène-2,2'-disulfonate de sodium, l'acide 4,4'-bis-[(4-anilino-6-bis(2-hydroxyéthyl)amino-1,3,5-triazin-2-yl)amino] stilbène-2,2'-disulfonique, le 4,4'-bis-[anilino-6-morpholine-1,3,5-triazin-2-yl)amino] stilbène, le 4,4'-bis-[(4-anilino-6-(2-hydroxyéthyl)méthyl amino-1,3,5-triazin-2-yl)amino]stilbène-2,2'- disodium sulfonate,
- le 2,5 thiophène di-yl bis(5 ter-butyl-1,3 benzoxazole),
- un dérivé anionique du di-aminostilbène en dispersion dans l'eau, et
- leurs mélanges.

43. Composition selon l'une quelconque des revendications 40 à 42, **caractérisée en ce que** l'azurant optique est choisi parmi le di-styryl-4,4' biphényle sulfonate di-sodique, le 4,4'-bis[4,6-diamilino-1,3,5-triazin-2-yl)amino]stilbène-2,2'-disulfonate de sodium, le 2,5 thiophène di-yl bis(5 ter-butyl-1,3 benzoxazole), et leurs mélanges.

44. Composition selon l'une quelconque des revendications précédentes, **caractérisée par le fait que** ladite matière colorante principale est présente en une teneur allant de 1 % à 80 % en poids, par rapport au poids total de la composition, de préférence allant de 1 % à 70 % en poids, de préférence allant de 1 % à 60 % en poids, préférentiellement allant de 5 % à 50 % en poids, plus préférentiellement allant de 5 % à 40 % en poids, et encore plus préférentiellement allant de 5 % à 30 % en poids.

45. Composition selon l'une quelconque des revendications précédentes, **caractérisée par le fait qu'**elle comprend une matière colorante additionnelle pulvérulente différente des matières colorantes décrites selon les revendications précédentes.

46. Composition selon la revendication précédente, **caractérisée par le fait que** la matière colorante additionnelle pulvérulente est choisie parmi les pigments et les nacres, différents des matières colorantes décrites selon les revendications précédentes.

47. Composition selon la revendication précédente, **caractérisée par le fait que** les pigments sont choisis parmi le dioxyde de titane, les oxydes de zirconium, les oxydes de cérium, les oxydes de zinc, les oxydes de fer, les oxydes de chrome, le violet de manganèse, le bleu outremer, l'hydrate de chrome, le bleu ferrique.

48. Composition selon l'une quelconque des revendications 46 et 47, **caractérisée par le fait que** les pigments sont présents en un teneur allant de 0.5 % à 30 % en poids, par rapport au poids de la composition, de préférence allant de 0,5 % à 20 % en poids.

49. Composition selon la revendication 46, **caractérisée par le fait que** les nacres sont choisies parmi le mica recouvert de titane, le mica recouvert d'oxychlorure de bismuth, le mica titane recouvert avec des oxydes de fer, le mica titane recouvert avec du bleu ferrique, le mica titane recouvert avec de l'oxyde de chrome, le mica titane recouvert avec un pigment organique, les pigments nacrés à base d'oxychlorure de bismuth.

50. Composition selon l'une quelconque des revendications 46 et 49, **caractérisée par le fait que** les nacres sont présentes en une teneur allant de 0,5 % à 50 % en poids, par rapport au poids total de la composition, de préférence allant de 0,5 % à 30 % en poids.

51. Composition selon l'une quelconque des revendications précédentes, **caractérisée par le fait qu'**elle comprend une charge additionnelle différente des particules solides ayant un indice de réfraction allant de 1,2 à 1,45.

52. Composition selon la revendication précédente, **caractérisée par le fait que** la charge additionnelle est choisie parmi le mica, le nitrure de bore, les particules de silice ayant indice de réfraction supérieur à 1,45, les poudres de polyamide, les poudres de polyéthylène, les poudres de copolymère diméthacrylate d'éthylèneglycol et de méthacrylate de lauryle, les particules de polyméthacrylate de méthyle, et leurs mélanges.

53. Composition selon la revendication 51 ou 52, **caractérisée par le fait que** la charge additionnelle est présente en une teneur allant de 0,5 % à 50 % en poids, par rapport au poids total de la composition, de préférence allant de 0,5 % à 30 % en poids, et préférentiellement allant de 0,5 % à 20% en poids.

54. Composition selon l'une quelconque des revendications précédentes, **caractérisée par le fait qu'**elle comprend une teneur totale en poudres additionnelles distinctes des particules solides ayant un indice de réfraction allant de 1,2 à 1,45 allant de 20 % à 80 % en poids, par rapport au poids total de la composition, de préférence allant de 30 % à 70 % en poids, préférentiellement allant de 35 % à 65 % en poids, et plus préférentiellement allant de 40 % à 60 % en poids.

55. Composition selon l'une quelconque des revendications précédentes, **caractérisée par le fait que** les particules solides d'organopolysiloxane réticulé ayant un indice de réfraction allant de 1,2 à 1,45 et les autres particules additionnelles distinctes des particules solides ayant un indice de réfraction allant de 1,2 à 1,45 sont présentes en des teneurs telles que le rapport pondéral particules d'organopolysiloxane réticulé/particules additionnelles va de 0,4/1 à 2,5/1, de préférence va de 0,4/1 à 2/1, préférentiellement va de 0,4/1 à 1,5/1, plus préférentiellement va de 0,4/1 à 1,3/1, et encore plus préférentiellement va de 0,6/1 à 1,3/1.

56. Composition selon l'une quelconque des revendications précédentes, **caractérisée par le fait qu'**elle comprend un ingrédient cosmétique choisi parmi les antioxydants, les parfums, les conservateurs, les fibres, les neutralisants, les tensioactifs, les cires, les filtres solaires, les vitamines, les hydratants, les composés auto-bronzants, les actifs antirides.

57. Composition selon l'une quelconque des revendications précédentes, **caractérisée par le fait que** la composition est sous la forme d'une poudre libre, d'une poudre compacte, d'un poudre pressée, d'une composition pâteuse.

58. Composition selon l'une quelconque des revendications précédentes, **caractérisée par le fait que** la composition est une composition solide.

59. Composition selon l'une quelconque des revendications précédentes, **caractérisée par le fait que** la composition est sous la forme d'un fond de teint, d'un fard à paupières, d'un fard à joue, d'un produit anticernes, d'un produit de maquillage du corps, d'un produit de maquillage des lèvres.

60. Procédé de maquillage de la peau ou des lèvres comprenant l'application sur la peau ou les lèvres d'une composition selon l'une quelconque des revendications précédentes.

## Claims

1. Powderous makeup composition comprising solid particles of elastomeric crosslinked organopolysiloxane having a refractive index ranging from 1.2 to 1.45, the said particles being in the form of an aqueous dispersion, a liquid binder having a refractive index ranging from 1.2 to 1.6 and a colorant selected from: reflective particles other than reflective particles comprising a mica substrate; goniochromatic colouring agents; photochromic colouring agents; fluorescent agents; and mixtures thereof.

2. Composition according to Claim 1, **characterized in that** the refractive index of the said solid particles ranges from 1.25 to 1.45, preferably from 1.3 to 1.45, preferentially from 1.35 to 1.45, more preferentially from 1.38 to 1.45 and more preferentially still from 1.40 to 1.45.

3. Composition according to either one of the preceding claims, **characterized in that** the crosslinked organopolysiloxane is selected from those obtained by:
- crosslinking addition reaction of a diorganosiloxane containing at least one hydrogen bonded to the silicon and a diorganopolysiloxane having ethylenically unsaturated groups bonded to the silicon;
- dehydrogenation crosslinking condensation reaction between a diorganopolysiloxane having hydroxyl end groups and a diorganopolysiloxane containing at least one hydrogen bonded to the silicon;
- crosslinking condensation reaction of a diorganopolysiloxane having hydroxyl end groups and a hydrolysable organopolysilane;
- thermal crosslinking of organopolysiloxane; and
- crosslinking of organopolysiloxane by high-energy radiation.

4. Composition according to any one of the preceding claims, **characterized in that** the crosslinked organopolysiloxane is obtained by crosslinking addition reaction (A) of a diorganopolysiloxane containing at least two hydrogens each bonded to a silicon and (B) of a diorganopolysiloxane having at least two ethylenically unsaturated groups bonded to silicon, particularly in the presence (C) of platinum catalyst.

5. Composition according to any one of the preceding claims, **characterized in that** the crosslinked organopolysiloxane is obtained by reacting a dimethylpolysiloxane having dimethylvinylsiloxy end groups and a methylhydrogenpolysiloxane having trimethylsiloxy end groups in the presence of a platinum catalyst.

6. Composition according to any one of the preceding claims, **characterized in that** the crosslinked organopolysiloxane is a non-emulsifying elastomer.

7. Composition according to any one of the preceding claims, **characterized in that** the said solid particles of elastomeric crosslinked organopolysiloxane having a refractive index ranging from 1.2 to 1.45 are present in an amount ranging from 5% to 80% by weight, relative to the total weight of the composition, preferably ranging from 10% to 70% by weight, more preferably ranging from 10% to 60% by weight, preferentially ranging from 10% to 50% by weight, more preferentially ranging from 15% to 50% by weight, even more preferentially ranging from 20% to 45% by weight and more preferentially still ranging from 20% to 40% by weight.

8. Composition according to any one of the preceding claims, **characterized in that** the liquid binder has a refractive index ranging from 1.2 to 1.5, preferably ranging from 1.25 to 1.45 and preferentially ranging from 1.3 to 1.45.

9. Composition according to any one of the preceding claims, **characterized in that** the liquid binder is selected from water, water-miscible organic solvents, oils and mixtures thereof.

10. Composition according to any one of the preceding claims, **characterized in that** the liquid binder is selected from water, water-miscible organic solvents and mixtures thereof.

11. Composition according to Claim 9 or 10, **characterized in that** the water-miscible organic solvents are selected from monoalcohols having from 2 to 6 carbon atoms; polyols having from 2 to 20 carbon atoms, preferably having from 2 to 10 carbon atoms, and preferentially having from 2 to 6 carbon atoms; glycol ethers having from 3 to 16 carbon atoms; and mixtures thereof.

12. Composition according to any one of Claims 9 to 12, **characterized in that** the water-miscible organic solvents are selected from ethanol, isopropanol, glycerol, propylene glycol, butylene glycol, pentylene glycol, hexylene glycol, dipropylene glycol, diethylene glycol, mono-, di- or tripropylene glycol (C₁-C₄)alkyl ethers, mono-, di- or triethylene glycol (C₁-C₄)alkyl ethers and mixtures thereof.

13. Composition according to Claim 9, **characterized in that** the oils are selected from hydrocarbon oils, silicone oils, fluoro oils and mixtures thereof.

14. Composition according to Claim 9 or 10, **characterized in that** the oils are selected from volatile oils.

15. Composition according to any one of the preceding claims, **characterized in that** the liquid binder is present in an amount ranging from 5o to 60% by weight, relative to the total weight of the composition, preferably ranging from 5% to 50% by weight, more preferably ranging from 5% to 45% by weight, preferentially ranging from 10% to 40% by weight and more preferentially ranging from 15% to 35% by weight.

16. Composition according to any one of the preceding claims, **characterized in that** the colorant is selected from reflective particles other than reflective particles comprising a mica substrate.

17. Composition according to any one of the preceding claims, **characterized in that** the reflective particles other than reflective particles comprising a mica substrate are selected from:
- particles comprising a natural or synthetic substrate coated at least partially with at least one layer of at least one metal,
- particles comprising a synthetic substrate, other than mica, coated at least partially with at least one layer of at least one metal compound, and particularly a metal oxide,
- particles formed from a stack of at least two layers of materials having different refractive indices, it being possible for at least one of these layers to be a polymer, and
- metal particles;
- and mixtures thereof.

18. Composition according to any one of the preceding claims, **characterized in that** the reflective particles are selected from particles comprising a substrate coated at least partially with at least one layer of at least one metal.

19. Composition according to the preceding claim, **characterized in that** the metal is selected from Ag, Au, Cu, Al, Zn, Ni, Mo, Cr and alloys thereof.

20. Composition according to the preceding claim, **characterized in that** the reflective particles are selected from particles comprising a synthetic substrate other than mica coated at least partially with at least one layer of at least one metal compound and in particular a metal oxide.

21. Composition according to the preceding claim, **characterized in that** the metal compound is selected from titanium oxides, especially TiO₂, iron oxides, tin oxides, especially Fe₂O₃, chromium oxides, barium sulphate and the compounds MgF₂, CeF₃, ZnS, ZnSe, SiO₂, Al₂O₃, MgO, Y₂O₃, SeO₃, SiO, HfO₂, ZrO₂, CeO₂, Nb₂O₅, Ta₂O₅ and MoS₂ and mixtures thereof.

22. Composition according to the preceding claim, **characterized in that** the metal compound is a titanium oxide, an iron oxide or a tin oxide or one of their mixtures.

23. Composition according to any one of Claims 20 to 22, **characterized in that** the synthetic substrate is selected from substrates comprising a single material or multiple materials, organic substrates, inorganic substrates, glasses, ceramics, metal oxides, aluminas, silicas and silicates, especially aluminosilicates and borosilicates.

24. Composition according to the preceding claim, **characterized in that** the synthetic substrate is selected from glasses.

25. Composition according to Claim 16, **characterized in that** the reflective particles are selected from particles formed by a stack of at least two layers of polymers having different refractive indices.

26. Composition according to any one of the preceding claims, **characterized in that** the reflective particles are selected from reflective metal particles.

27. Composition according to the preceding claim, **characterized in that** the reflective metal particles are selected from particles of gold, silver, aluminium and copper.

28. Composition according to any one of the preceding claims, **characterized in that** it comprises goniochromatic colouring agents.

29. Composition according to the preceding claim, **characterized in that** the goniochromatic colouring agent has a multi-layer interference structure.

30. Composition according to the preceding claim, **characterized in that** the goniochromatic colouring agents are pigments having a multi-layer interference structure comprising at least two layers, each layer being made of at least one material selected from the group consisting of the following materials: MgF₂, CeF₃, ZnS, ZnSe, Si, SiO₂, Ge, Te, Fe₂O₃, Pt, Va, Al₂O₃, MgO, Y₂O₃, S₂O₃, SiO, HeO₂, ZrO₂ , CeO₂ , Nib₂O₅, Ta₂O₅, TiO₂, Ag, Al, Au, Cr, Cu, Rb, Ti, Ta, W, Zn, MoS₂, cryolite, alloys, polymers and combinations thereof.

31. Composition according to either of Claims 29 and 30, wherein the goniochromatic colouring agents are pigments having a multi-layer interference structure selected from the group consisting of the following structures: Al/SiO₂/Al/SiO₂/Al; Cr/MgF_{2/}Al/MgF2/Cr; MoS₂/SiO₂/Al/SiO₂/MoS₂; Fe₂O₃/SiO₂/Al/SiO₂/Fe₂O₃; Fe₂O₃/SiO₂/Fe₂O₃/SiO₂/Fe₂O₃; MoS₂/SiO₂/mica-oxide/SiO₂/MoS₂; Fe₂O₃/SiO₂/mica oxide/SiO₂/Fe₂O₃ and combinations thereof.

32. Composition according to Claim 28, **characterized in that** the goniochromatic colouring agent is a liquid-crystal colouring agent.

33. Composition according to the preceding claim, **characterized in that** the liquid-crystal goniochromatic colouring agent is obtained by polymerizing a mixture of monomers comprising:
- a) at least one first monomer A of formula (I) Y1-A1-M1-A2-Y2
in which
- i) Y1 and Y2, which are identical or different, represent an acrylate or methacrylate group, preferably an acrylate group;
- ii) A1 and A2, which are identical or different, represent a group of formula -CₙH₂ₙ-, in which n is an integer ranging from 1 to 20, preferably ranging from 2 to 6 and in particular equal to 4;
- iii) M1 denotes a group of general formula (I')
-R₁-X₁-R₂-X₂-R₃-X₃-R₄-, in which R₁ and R₄ denote -O-, R₂ and R₃ denote -COO- and X₁, X₂ and X₃ are a 1,4-phenylene group, the carbonyl group -CO- respectively of R₂ and of R₃ being bonded respectively to the X₁ group and to the X₃ group,
and
- b) at least one chiral second monomer B of formula (II) V1-W1-Z-W2-V2, in which
- i) V1 denotes an acrylate or methacrylate group, and in particular an acrylate group, and V2 denotes a C₁-C₂₀ alkyl group, a C₁-C₂₀ alkoxy, a (C₁-C₂₀) alkoxycarbonyl or -OH and preferably denotes a C₁-C₂₀ alkoxy group, in particular a C₁-C₄ alkoxy group and especially a methoxy group;
- ii) W1 represents a divalent group of formula -X'1-CO-O-,
W2 represents a divalent group of formula -O-CO-X'1-, in which formulae X'1 denotes a 1,4-phenylene group, and Z denotes a chiral group comprising two bonds, originating from the dianhydrohexitol group, in particular a divalent radical of formula:

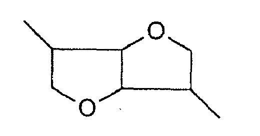

34. Composition according to the preceding claim, **characterized in that** the liquid-crystal colouring agent is obtained from a mixture of monomers comprising from 70% to 99% by weight of monomer A and from 1% to 30% by weight of monomer B, relative to the total weight of monomer A and of monomer B, and in particular comprising from 90% to 95% by weight of monomer A and from 5% to 10% by weight of monomer B.

35. Composition according to any one of the preceding claims, **characterized in that** it comprises a photochromic colouring agent.

36. Composition according to any one of the preceding claims, **characterized in that** the photochromic colouring agent is selected from spiro-oxazines and derivatives thereof, spironaphthoxazines, naphthopyran and its derivatives, spiropyrans, nitrobenzylpyridines, spirolanes, titanium oxide or zinc oxide doped with iron, aluminosilicates doped with metal ions or with a group selected from Se, S, OH, SO₄²⁻ and WO₄²⁻.

37. Composition according to any one of the preceding claims, **characterized in that** it comprises a fluorescent agent.

38. Composition according to the preceding claim, **characterized in that** the fluorescent agent is selected from organic fluorescent pigments.

39. Composition according to the preceding claim, **characterized in that** the said fluorescent pigments are selected from coloured polyamide resins and/or formaldehyde/benzoguanamine resins and/or melamine/formaldehyde/sulphonamide resins, from coloured aminotriazine/formaldehyde/sulphonamide co-condensates and/or from metallized polyester flakes and/or mixtures thereof.

40. Composition according to Claim 37, **characterized in that** the fluorescent agent is selected from optical brighteners.

41. Cosmetic composition according to the preceding claim, **characterized in that** the optical brightener is selected from stilbene derivatives, coumarin derivatives, oxazole, benzoxazole, imidazole and triazole derivatives, pyrazolines, pyrene derivatives, porphyrin derivatives and mixtures thereof.

42. Composition according to Claim 40 or 41, **characterized in that** the optical brightener is selected from:
- the stilbene derivative of naphthotriazole, disodium 4,4'-distyrylbiphenylsulphonate (CTFA name: disodium distyrylbiphenyl disulphonate), sodium 4,4'-bis[(4,6-dianilino-1,3,5-triazin-2-yl)-amino]stilbene-2,2'-disulphonate, 4,4'-bis-[(4-anilino-6-bis(2-hydroxyethyl)amino-1,3,5-triazin-2-yl)amino]-stilbene-2,2'-disulphonic acid, 4,4'-bis[(4-anilino-6-morpholine-1,3,5-triazin-2-yl)amino]-stilbene, disodium 4,4'-bis-[(4-anilino-6-(2-hydroxyethyl)methyl amino-1,3,5-triazin-2-yl)amino]stilbene-2,2'-disulphonate,
- 2,5-thiophenediylbis(5-tert-butyl-1,3-benzoxazole),
- an anionic derivative of diaminostilbene in dispersion in water, and
- mixtures thereof.

43. Composition according to any one of Claims 40 to 42, **characterized in that** the optical brightener is selected from disodium 4,4'-distyrylbiphenylsulphonate, sodium 4,4'-bis[4,6-diamilino-1,3,5-triazin-2-yl)amino]stilbene-2,2'-disulphonate, 2,5-thiophenediylbis(5-tert-butyl-1,3 benzoxazole), and mixtures thereof.

44. Composition according to any one of the preceding claims, **characterized in that** the said principal colorant is present in an amount ranging from 1% to 80% by weight, relative to the total weight of the composition, preferably ranging from 1% to 70% by weight, preferably ranging from 1% to 60% by weight, preferentially ranging from 5% to 50% by weight, more preferentially ranging from 5% to 40% by weight and more preferentially still ranging from 5% to 30% by weight.

45. Composition according to any one of the preceding claims, **characterized in that** it comprises an additional pulverulent colorant other than the colorants described according to the preceding claims.

46. Composition according to the preceding claim, **characterized in that** the additional pulverulent colorant is selected from pigments and nacres other than the colorants described according to the preceding claims.

47. Composition according to the preceding claim, **characterized in that** the pigments are selected from titanium dioxide, zirconium oxides, cerium oxides, zinc oxides, iron oxides, chromium oxides, manganese violet, ultramarine blue, chromium hydrate and Prussian blue.

48. Composition according to either of Claims 46 and 47, **characterized in that** the pigments are present in an amount ranging from 0.5% to 30% by weight, relative to the weight of the composition, preferably ranging from 0.5% to 20% by weight.

49. Composition according to Claim 46, **characterized in that** the nacres are selected from mica covered with titanium, mica covered with bismuth oxychloride, titanium mica covered with iron oxides, titanium mica covered with Prussian blue, titanium mica covered with chromium oxide, titanium mica covered with an organic pigment, and nacreous pigments based on bismuth oxychloride.

50. Composition according to either of Claims 46 and 49, **characterized in that** the nacres are present in an amount ranging from 0.5% to 50% by weight, relative to the total weight of the composition, preferably ranging from 0.5% to 30% by weight.

51. Composition according to any one of the preceding claims, **characterized in that** it comprises an additional filler other than solid particles having a refractive index ranging from 1.2 to 1.45.

52. Composition according to the preceding claim, **characterized in that** the additional filler is selected from mica, boron nitride, silica particles having a refractive index of more than 1.45, polyamide powders, polyethylene powders, ethylene glycol dimethacrylate-lauryl methacrylate copolymer powders, polymethyl methacrylate particles and mixtures thereof.

53. Composition according to Claim 51 or 52, **characterized in that** the additional filler is present in an amount ranging from 0.5% to 50% by weight, ) relative to the total weight of the composition, preferably ranging from 0.5% to 30% by weight and preferentially ranging from 0.5% to 20% by weight.

54. Composition according to any one of the preceding claims, **characterized in that** it comprises a total amount of additional powders, other than the solid particles having a refractive index ranging from 1.2 to 1.45, ranging from 20% to 80% by weight, relative to the total weight of the composition, preferably ranging from 30% to 70% by weight, preferentially ranging from 35% to 65% by weight and more preferentially ranging from 40% to 60% by weight.

55. Composition according to any one of the preceding claims, **characterized in that** the solid particles of crosslinked organopolysiloxane having a refractive index ranging from 1.2 to 1.45 and the other, additional particles, other than the solid particles having a refractive index ranging from 1.2 to 1.45, are present in amounts such that the crosslinked organopolysiloxane particles/additional particles' weight ratio is from 0.4/1 to 2.5/1, preferably from 0.4/1 to 2/1, preferentially from 0.4/1 to 1.5/1, more preferentially from 0.4/1 to 1.3/1, and even more preferentially from 0.6/1 to 1.3/1.

56. Composition according to any one of the preceding claims, **characterized in that** it comprises a cosmetic ingredient selected from antioxidants, perfumes, preservatives, fibres, neutralizing agents, surfactants, waxes, sunscreens, vitamins, moisturizers, self-tanning compounds and anti-wrinkle actives.

57. Composition according to any one of the preceding claims, **characterized in that** the composition is in the form of a loose powder, a compact powder, a pressed powder or a pasty composition.

58. Composition according to any one of the preceding claims, **characterized in that** the composition is a solid composition.

59. Composition according to any one of the preceding claims, **characterized in that** the composition is in the form of a foundation, an eyeshadow, a blusher, a concealer product, a body makeup product or a lip makeup product.

60. Method of making up the skin or lips, comprising applying to the skin or to the lips a composition according to any one of the preceding claims.

## Patentansprüche

1. Pulverförmige Zusammensetzung zum Schminken, die feste Partikel eines vernetzten elastomeren Organopolysiloxans mit einer Brechzahl im Bereich von 1,2 bis 1,45, wobei die Partikel in der Form einer wässrigen Dispersion vorliegen, ein flüssiges Bindemittel mit einer Brechzahl im Bereich von 1,2 bis 1,6 und ein Farbmittel enthält, das unter den reflektierenden Partikeln, die von den ein Glimmersubstrat enthaltenden reflektierenden Partikeln verschieden sind, goniochromatischen Farbmitteln, photochromen Farbmitteln, fluoreszierenden Stoffen und deren Gemischen ausgewählt ist.

2. Zusammensetzung nach Anspruch 1, **dadurch gekennzeichnet, dass** die Brechzahl der festen Partikel im Bereich von 1,25 bis 1,45, vorzugsweise im Bereich von 1,3 bis 1,45, bevorzugt im Bereich von 1,35 bis 1,45, noch bevorzugter im Bereich von 1,38 bis 1,45 und besonders bevorzugt im Bereich von 1,40 bis 1,45 liegt.

3. Zusammensetzung nach einem der vorhergehenden Ansprüche, **dadurch gekennzeichnet, dass** das vernetzte Organopolysiloxan unter den Verbindungen ausgewählt ist, die erhalten werden:
- durch vernetzende Addition eines Diorganosiloxans, das mindestens einen an Silicium gebundenen Wasserstoff aufweist, und eines Diorganopolysiloxans, das an Silicium gebundene Gruppen mit ethylenisch ungesättigter Bindung aufweist;
- durch vernetzende Kondensation (Dehydrierung) eines Diorganopolysiloxans mit endständigen Hydroxygruppen und eines Diorganopolysiloxans, das mindestens einen an Silicium gebundenen Wasserstoff aufweist;
- durch vernetzende Kondensation eines Diorganopolysiloxans mit endständigen Hydroxygruppen und eines hydrolysierbaren Organopolysiloxans;
- durch thermische Vernetzung eines Organopolysiloxans;
- durch Vernetzung eines Organopolysiloxans mit hochenergetischer Strahlung.

4. Zusammensetzung nach einem der vorhergehenden Ansprüche, **dadurch gekennzeichnet, dass** das vernetzte Organopolysiloxan durch vernetzende Addition (A) eines Diorganopolysiloxans, das mindestens zwei jeweils an Silicium gebundene Wasserstoffe aufweist, und (B) eines Diorganopolysiloxans, das mindestens zwei an Silicium gebundene Gruppen mit ethylenisch ungesättigter Bindung aufweist, insbesondere in Gegenwart (C) eines Platinkatalysators erhalten wird.

5. Zusammensetzung nach einem der vorhergehenden Ansprüche, **dadurch gekennzeichnet, dass** das vernetzte Organopolysiloxan durch Umsetzung eines Dimethylpolysiloxans mit endständigen Dimethylvinylsiloxygruppen und eines Methylhydrogenopolysiloxans mit endständigen Trimethylsiloxygruppen in Gegenwart eines Platinkatalysators gebildet wird.

6. Zusammensetzung nach einem der vorhergehenden Ansprüche, **dadurch gekennzeichnet, dass** das vernetzte Organopolysiloxan ein nicht emulgierendes Elastomer ist.

7. Zusammensetzung nach einem der vorhergehenden Ansprüche, **dadurch gekennzeichnet, dass** die festen Partikel eines vernetzten elastomeren Organopolysiloxans mit einer Brechzahl im Bereich von 1,2 bis 1,45 in einem Mengenanteil im Bereich von 5 bis 80 Gew.-%, bezogen auf das Gesamtgewicht der Zusammensetzung, vorzugsweise im Bereich von 10 bis 70 Gew.- %, bevorzugt im Bereich von 10 bis 60 Gew.-%, vorzugsweise im Bereich von 10 bis 50 Gew.-%, noch bevorzugter im Bereich von 15 bis 50 Gew.-%, noch bevorzugter im Bereich von 20 bis 45 Gew.-% und besonders bevorzugt im Bereich von 20 bis 40 Gew.- % enthalten sind.

8. Zusammensetzung nach einem der vorhergehenden Ansprüche, **dadurch gekennzeichnet, dass** das flüssige Bindemittel eine Brechzahl im Bereich von 1,2 bis 1,5, vorzugsweise 1,25 bis 1,45 und bevorzugt 1,3 bis 1,45 aufweist.

9. Zusammensetzung nach einem der vorhergehenden Ansprüche, **dadurch gekennzeichnet, dass** das flüssige Bindemittel unter Wasser, mit Wasser mischbaren organischen Lösemitteln, Ölen und deren Gemischen ausgewählt ist.

10. Zusammensetzung nach einem der vorhergehenden Ansprüche, **dadurch gekennzeichnet, dass** das flüssige Bindemittel unter Wasser, mit Wasser mischbaren organischen Lösemitteln und deren Gemischen ausgewählt ist.

11. Zusammensetzung nach Anspruch 9 oder 10, **dadurch gekennzeichnet, dass** die mit Wasser mischbaren organischen Lösemittel unter den Monoalkoholen mit 2 bis 6 Kohlenstoffatomen; Polyolen mit 2 bis 20 Kohlenstoffatomen, vorzugsweise 2 bis 10 Kohlenstoffatomen und bevorzugt 2 bis 6 Kohlenstoffatomen ; Glycolethern mit 3 bis 16 Kohlenstoffatomen und deren Gemischen ausgewählt sind.

12. Zusammensetzung nach einem der Ansprüche 9 bis 12, **dadurch gekennzeichnet, dass** die mit Wasser mischbaren organischen Lösemittel unter Ethanol, Isopropanol, Glycerin, Propylenglycol, Butylenglycol, Pentylenglycol, Hexylenglycol, Dipropylenglycol, Diethylenglycol, Alkyl(C₁₋₄)ethern von Mono-, Di- oder Tripropylenglycol, Alkyl(C₁₋₄)ethern von Mono-, Di- oder Triethylenglycol und deren Gemischen ausgewählt sind.

13. Zusammensetzung nach Anspruch 9, **dadurch gekennzeichnet, dass** die Öle unter den Kohlenwasserstoffölen, Siliconölen. fluorierten Ölen und deren Gemischen ausgewählt sind.

14. Zusammensetzung nach Anspruch 9 oder 10, **dadurch gekennzeichnet, dass** die Öle unter den flüchtigen Ölen ausgewählt sind.

15. Zusammensetzung nach einem der vorhergehenden Ansprüche, **dadurch gekennzeichnet, dass** das flüssige Bindemittel in einem Mengenanteil von 5 bis 60 Gew.-%, bezogen auf das Gesamtgewicht der Zusammensetzung, vorzugsweise 5 bis 50 Gew.-%, bevorzugt 5 bis 45 Gew.-%, vorzugsweise 10 bis 40 Gew.-% und noch bevorzugter 15 bis 35 Gew.-% enthalten ist.

16. Zusammensetzung nach einem der vorhergehenden Ansprüche, **dadurch gekennzeichnet, dass** das Farbmittel unter den reflektierenden Partikeln, die von den ein Glimmersubstrat enthaltenden reflektierenden Partikel verschieden sind, ausgewählt ist.

17. Zusammensetzung nach einem der vorhergehenden Ansprüche, **dadurch gekennzeichnet, dass** die reflektierenden Partikeln, die von den ein Glimmersubstrat enthaltenden reflektierenden Partikel verschieden sind, ausgewählt sind unter:
- Partikeln mit einem natürlichen oder synthetischen Substrat, das zumindest zum Teil mit zumindest einer Schicht mindestens eines Metalls umhüllt ist;
- Partikeln mit einem von Glimmer verschiedenen, synthetischen Substrat, das zumindest zum Teil mit zumindest einer Schicht mindestens einer Metallverbindung und insbesondere eines Metalloxids umhüllt ist;
- Partikeln, die aus einem Stapel mindestens zweier Schichten aus Materialien mit unterschiedlichen Brechungsindices gebildet sind, wobei mindestens eine dieser Schichten ein Polymer sein kann; und
- Metallpartikeln; und
- deren Gemischen.

18. Zusammensetzung nach einem der vorhergehenden Ansprüche, **dadurch gekennzeichnet, dass** die reflektierenden Partikel unter den Partikeln mit einem Substrat ausgewählt sind, das zumindest zum Teil mit zumindest einer Schicht mindestens eines Metalls umhüllt ist.

19. Zusammensetzung nach dem vorhergehenden Anspruch, **dadurch gekennzeichnet, dass** das Metall unter Ag, Au, Cu, Al, Zn, Ni, Mo, Cr und deren Legierungen ausgewählt ist.

20. Zusammensetzung nach dem vorhergehenden Anspruch, **dadurch gekennzeichnet, dass** die reflektierenden Partikel unter den Partikeln mit einem von Glimmer verschiedenen, synthetischen Substrat ausgewählt sind, das zumindest zum Teil mit zumindest einer Schicht mindestens einer Metallverbindung und insbesondere eines Metalloxids umhüllt ist.

21. Zusammensetzung nach dem vorhergehenden Anspruch, **dadurch gekennzeichnet, dass** die Metallverbindung unter den Oxiden von Titan und insbesondere TiO₂, von Eisen, von Zinn, insbesondere Fe₂O₃, von Chrom, Bariumsulfat und den Verbindungen MgF₂, CeF₃, ZnS, ZnSe, SiO₂, Al₂O₃, MgO, Y₂O₃, SeO₃, SiO, HfO₂, ZrO₂, CeO₂, Nb₂O₅, Ta₂O₅ und MoS₂ und deren Gemischen ausgewählt ist.

22. Zusammensetzung nach dem vorhergehenden Anspruch, **dadurch gekennzeichnet, dass** die Metallverbindung ein Titanoxid, ein Eisenoxid oder ein Zinnoxid oder eines ihrer Gemische ist.

23. Zusammensetzung nach einem der Ansprüche 20 bis 22, **dadurch gekennzeichnet, dass** das synthetische Substrat unter den aus einem Material bestehenden Substraten, Multimaterialien, organischen Substraten, anorganischen Substraten, Gläsern, Keramiken, Metalloxiden, Aluminiumoxiden, Kieselsäuren, Silicaten und insbesondere Aluminosilicaten und Borsilicaten ausgewählt ist.

24. Zusammensetzung nach dem vorhergehenden Anspruch, **dadurch gekennzeichnet, dass** das synthetische Substrat unter den Gläsern ausgewählt ist.

25. Zusammensetzung nach Anspruch 16, **dadurch gekennzeichnet, dass** die reflektierenden Partikel unter den Partikeln ausgewählt sind, die aus einem Stapel mindestens zweier Schichten aus Materialien mit unterschiedlichen Brechungsindices gebildet ist.

26. Zusammensetzung nach einem der vorhergehenden Ansprüche, **dadurch gekennzeichnet, dass** die reflektierenden Partikel unter den reflektierenden Metallpartikeln ausgewählt sind.

27. Zusammensetzung nach dem vorhergehenden Anspruch, **dadurch gekennzeichnet, dass** die reflektierenden Metallpartikel unter den Goldpartikeln, Silberpartikeln, Aluminiumpartikeln und Kupferpartikeln ausgewählt sind.

28. Zusammensetzung nach einem der vorhergehenden Ansprüche, **dadurch gekennzeichnet, dass** sie goniochromatische Farbmittel enthält.

29. Zusammensetzung nach dem vorhergehenden Anspruch, **dadurch gekennzeichnet, dass** das goniochromatische Farbmittel eine interferierende Mehrschichtstruktur aufweist.

30. Zusammensetzung nach dem vorhergehenden Anspruch, **dadurch gekennzeichnet, dass** die goniochromatischen Farbmittel Pigmente mit einer Interferenz-Mehrschichtstruktur sind, die mindestens zwei Schichten aufweist, wobei jede Schicht aus mindestens einem Material realisiert ist, das unter den folgenden Materialien ausgewählt ist: MgF₂, CeF₃, ZnS, ZnSe, Si, SiO₂, Ge, Te, Fe₂O₃, Pt, Va, Al₂O₃, MgO, Y₂O₃, S₂O₃, SiO, HfO₂, ZrO₂, CeO₂, Nb₂O₅, Ta₂O₅, TiO₂, Ag, Al, Au, Cr, Cu, Rb, Ti, Ta, W, Zn, MoS₂, Kryolith, Legierungen, Polymeren und deren Kombinationen.

31. Zusammensetzung nach einem der Ansprüche 29 oder 30, wobei die goniochromatischen Farbmittel Pigmente mit einer Interferenz-Mehrschichtstruktur sind, die unter den folgenden Strukturen ausgewählt ist: Al/SiO₂/Al/SiO₂/Al; Cr/MgF₂/Al/MgF₂/Cr; MoS₂/SiO₂/Al/SiO₂/MoS₂; Fe₂O₃/SiO₂/Al/SiO₂/Fe₂O₃; Fe₂O₃/SiO₂/Fe₂O₃/SiO₂/Fe₂O₃; MoS₂/SiO₂/Glimmeroxid/SiO₂/MoS₂; Fe₂O₃/SiO₂/Glimmeroxid/SiO₂/Fe₂O₃ und ihren Kombinationen.

32. Zusammensetzung nach Anspruch 28, **dadurch gekennzeichnet, dass** das goniochromatische Farbmittel ein Farbmittel mit Flüssigkristallen ist.

33. Zusammensetzung nach dem vorhergehenden Anspruch, **dadurch gekennzeichnet, dass** das flüssigkristalline goniochromatische Farbmittel durch Polymerisation eines Gemisches von Monomeren erhalten wird, das aufweist:
a) mindestens ein erstes Monomer A der Formel (I) Y1-A1-M1-A2-Y2, wobei in der Formel:
(i) Y1 und Y2, die gleich oder verschieden sind, bedeuten eine Acrylatgruppe oder eine Methacrylatgruppe, vorzugsweise eine Acrylatgruppe;
(ii) A1 und A2, die gleich oder verschieden sind, bedeuten eine Gruppe der Formel -CnH₂n-, worin n eine ganze Zahl im Bereich von 1 bis 20, insbesondere im Bereich von 2 bis 6 und besonders 4 bedeutet;
(iii) M1 bedeutet eine Gruppe der allgemeinen Formel (I') -R₁-X₁-R₂-X₂-R₃-X₃-R₄-, wobei R₁ und R₄ -O- und R₂ und R₃ -COO- bedeuten,
und X₁, X₂, X₃ eine 1,4-Phenylengruppe bedeuten, wobei die Carbonylgruppe -CO- von R₂ bzw. R₃ an die Gruppe X₁ bzw. X₃ gebunden ist; und
b) mindestens ein zweites chirales Monomer B der Formel (II) V1-W1-Z-W2-V2, wobei in der Formel:
(i) V1 bedeutet eine Acrylatgruppe oder eine Methacrylatgruppe und insbesondere eine Acrylatgruppe, und V2 bedeutet eine Alkyl(C₁₋₂₀)gruppe, eine Alkoxy(C₁₋₂₀)gruppe, eine Alkoxy(C₁₋₂₀)carbonylgruppe, -OH und vorzugsweise bedeutet V2 eine Alkoxy(C₁₋₂₀)gruppe, insbesondere eine Alkoxy(C₁₋₂₀)gruppe und besonders Methoxy;
(ii) W2 bedeutet eine zweiwertige Gruppe der Formel -X'1-CO-O-,
W2 bedeutet eine zweiwertige Gruppe der Formel -O-CO- X'1-, wobei X'1 eine 1,4-Phenylengruppe bedeutet,
und Z bedeutet eine chirale Gruppe mit zwei Bindungen, die von der Gruppe Dianhydrohexit stammt, und insbesondere eine zweiwertige Gruppe der Formel:

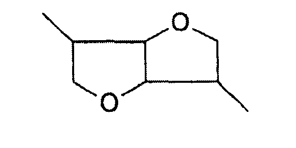

34. Zusammensetzung nach dem vorhergehenden Anspruch, **dadurch gekennzeichnet, dass** das Farbmittel mit Flüssigkristallen ausgehend von einem Monomerengemisch gebildet wird, das 70 bis 99 Gew-.% Monomer A und 1 bis 30 Gew.-% Monomer B, bezogen auf das Gesamtgewicht des Monomers A und des Monomers B, und insbesondere 90 bis 95 Gew.-% Monomer A und 5 bis 10 Gew.-% Monomer B enthält.

35. Zusammensetzung nach einem der vorhergehenden Ansprüche, **dadurch gekennzeichnet, dass** sie ein photochromes Farbmittel enthält.

36. Zusammensetzung nach einem der vorhergehenden Ansprüche, **dadurch gekennzeichnet, dass** das photochrome Farbmittel unter Spirooxazinen und ihren Derivaten, Spironaphthoxazinen, Naphthopyran und seinen Derivaten, Spiropyranen, Nitrobenzylpyridinen, Spirolanen, Oxiden von Titan oder Zink, die mit Eisen dotiert sind, oder Aluminosilicaten ausgewählt ist, die mit Metallionen oder einer Gruppe dotiert sind, die unter Se, S, OH, SO₄²⁻ und WO₄²⁻ ausgewählt ist.

37. Zusammensetzung nach einem der vorhergehenden Ansprüche, **dadurch gekennzeichnet, dass** sie einen fluoreszierenden Stoff enthält.

38. Zusammensetzung nach dem vorhergehenden Anspruch, **dadurch gekennzeichnet, dass** der fluoreszierende Stoff unter den fluoreszierenden organischen Pigmenten ausgewählt ist.

39. Zusammensetzung nach dem vorhergehenden Anspruch, **dadurch gekennzeichnet, dass** die fluoreszierenden Pigmente unter den farbigen Polyamidharzen und/oder Formaldehyd/Benzoguanamin-Harzen und/oder Melanin/Formaldehyd/Sulfonamid-Harzen, unter den farbigen Aminotriazin/ Formaldehyd/ Sulfonamid-Cokondensaten und/oder metallisierten Polyester-Pailletten und/oder deren Gemischen ausgewählt sind.

40. Zusammensetzung nach Anspruch 37, **dadurch gekennzeichnet, dass** der fluoreszierende Stoff unter den optischen Aufhellern ausgewählt ist.

41. Zusammensetzung nach dem vorhergehenden Anspruch, **dadurch gekennzeichnet, dass** der optische Aufheller unter den Stilbenderivaten, Cumarinderivaten, Oxazolderivaten, Benzoxazolderivaten, Imidazolderivaten, Triazolderivaten, Pyrazolinen, Pyrenderivaten, Porphyrinderivaten und deren Gemischen ausgewählt sind.

42. Zusammensetzung nach Anspruch 40 oder 41, **dadurch gekennzeichnet, dass** der optische Aufheller ausgewählt ist untdem Stilbenderivat von Naphthotriazol, Dinatrium-distyryl-4,4'-biphenylsulfonat (CTFA-Name: Disodium Distyrylbiphenyl Disulfonate), Dinatrium-4,4'-bis[(4,6-Dianilino-1,3,5-triazin-2-yl)amino]-stilben-2,2'-disulfonat, 4,4'-Bis[(4-anilino-6-bis(2-hydroxyethyl)amino-1,3,5-triazin-2-yl)amino]-stilben-2,2'-disulfonsäure, 4,4'-Bis[anilino-6-morpholin-1,3,5-triazin-2-yl)amino]-stilben, Dinatrium-4,4'-bis[(4-anilino-6-(2-hydroxyethyl)methyl-amino-1,3,5-triazin-2-yl)amino]stilben-2,2'-sulfonat,
- 2,5-Thiophen-diyl-bis(5-tert-butyl-1,3-benzoxazol),
- einem anionischen Derivat von Diaminostilben in Dispersion in Wasser, und
- deren Gemischen.

43. Zusammensetzung nach einem der Ansprüche 40 bis 42, **dadurch gekennzeichnet, dass** der optische Aufheller unter Dinatrium-distyryl-4,4'-biphenylsulfonat, Natrium-4,4'-bis[(4,6-Dianilino-1,3,5-triazin-2-yl)amino]-stilben-2,2'-disulfonat, 2,5-Thiophen-diyl-bis(5-tert-butyl-1,3-benzoxazol) und deren Gemischen ausgewählt ist.

44. Zusammensetzung nach einem der vorhergehenden Ansprüche, **dadurch gekennzeichnet, dass** das Hauptfarbmittel in einem Mengenanteil von 1 bis 80 Gew.-%, bezogen auf das Gesamtgewicht der Zusammensetzung, vorzugsweise 1 bis 70 Gew.-%, bevorzugt 1 bis 60 Gew.-%, vorzugsweise 5 bis 50 Gew.-%, noch bevorzugter 5 bis 40 Gew.-% und besonders bevorzugt 5 bis 30 Gew.-% enthalten ist.

45. Zusammensetzung nach einem der vorhergehenden Ansprüche, **dadurch gekennzeichnet, dass** sie ein ergänzendes pulverförmiges Farbmittel enthält, das von den in den vorhergehenden Ansprüchen beschriebenen Farbmitteln verschieden ist.

46. Zusammensetzung nach dem vorhergehenden Anspruch, **dadurch gekennzeichnet, dass** sie ein ergänzendes pulverförmiges Farbmittel enthält, das unter den Pigmenten und Perlglanzpigmenten ausgewählt ist, die von den in den vorhergehenden Ansprüchen beschriebenen Farbmitteln verschieden sind.

47. Zusammensetzung nach dem vorhergehenden Anspruch, **dadurch gekennzeichnet, dass** die Pigmente unter Titandioxid, Zirconiumoxiden, Ceroxiden, Zinkoxiden, Eisenoxiden, Chromoxiden, Manganviolett, Ultramarinblau, Chromhydrat und Eisenblau ausgewählt sind.

48. Zusammensetzung nach einem der Ansprüche 46 und 47, **dadurch gekennzeichnet, dass** die Pigmente in einem Mengenanteil von 0,5 bis 30 Gew.-%, bezogen auf das Gesamtgewicht der Zusammensetzung, und vorzugsweise 0,5 bis 20 Gew.-% vorliegen.

49. Zusammensetzung nach Anspruch 46, **dadurch gekennzeichnet, dass** die Perlglanzpigmente unter den mit Titan bedeckten Glimmerpigmenten, mit Bismutoxidchlorid bedeckten Glimmerpigmenten, mit Eisenoxiden bedeckten Glimmerpigmenten, mit Bismutoxidchlorid bedeckten Glimmerpigmenten, mit Eisenblau bedeckten Glimmerpigmenten, mit Chromoxid bedeckten Glimmerpigmenten, mit einem organischen Pigment bedeckten Glimmerpigmenten und Perlglanzpigmenten auf der Basis von Bismutoxidchlorid ausgewählt sind.

50. Zusammensetzung nach einem der Ansprüche 46 und 49, **dadurch gekennzeichnet, dass** die Perlglanzpigmente in einem Mengenanteil von 0,5 bis 50 Gew.-%, bezogen auf das Gesamtgewicht der Zusammensetzung, und vorzugsweise 0,5 bis 30 Gew.-% vorliegen.

51. Zusammensetzung nach einem der vorhergehenden Ansprüche, **dadurch gekennzeichnet, dass** sie einen ergänzenden Füllstoff enthält, der von den festen Partikeln mit einer Brechzahl von 1,2 bis 1,45 verschieden ist.

52. Zusammensetzung nach dem vorhergehenden Anspruch, **dadurch gekennzeichnet, dass** der zusätzliche Füllstoff unter Glimmer, Bornitrid, Kieselsäurepartikeln mit einer Brechzahl über 1,45, Polyamidpulvern, Polyethylenpulvern, Pulvern aus Ethylenglycoldimethacrylat/ Laurylmethacrylat-Copolymer, Partikeln aus Polymethylmethacrylat und deren Gemischen ausgewählt ist.

53. Zusammensetzung nach Anspruch 51 oder 52, **dadurch gekennzeichnet, dass** der zusätzliche Füllstoff in einem Mengenanteil von 0,5 bis 50 Gew.-%, bezogen auf das Gesamtgewicht der Zusammensetzung, vorzugsweise 0,5 bis 30 Gew.-% und bevorzugt 0,5 bis 20 Gew.-% vorliegt.

54. Zusammensetzung nach einem der vorhergehenden Ansprüche, **dadurch gekennzeichnet, dass** sie einen Gesamtgehalt an ergänzenden Pulvern, die von den festen Partikeln mit einer Brechzahl von 1,2 bis 1,45 verschieden sind, im Bereich von 20 bis 80 Gew.-%, bezogen auf das Gesamtgewicht der Zusammensetzung, vorzugsweise 30 bis 70 Gew.-%, bevorzugt 35 bis 65 Gew.-% und noch bevorzugter 40 bis 60 Gew.-% aufweist.

55. Zusammensetzung nach einem der vorhergehenden Ansprüche, **dadurch gekennzeichnet, dass** die festen Partikel eines vernetzten Polyorganosiloxans mit einer Brechzahl von 1,2 bis 1,45 und die weiteren ergänzenden Partikel, die von den festen Partikeln mit einer Brechzahl von 1,2 bis 1,45 verschieden sind, in einem solchen Mengenanteil enthalten sind, dass das Gewichtsverhältnis Partikel eines vernetzten Organopolysiloxans/ergänzende Partikel im Bereich von 0,4/1 bis 2,5/1, bevorzugt im Bereich von 0,4/1 bis 2/1, vorzugsweise im Bereich von 0,4/1 bis 1,5/1, noch bevorzugter 0,4/1 bis 1,3/1 und besonders bevorzugt 0,6/1 bis 1,3/1 liegt.

56. Zusammensetzung nach einem der vorhergehenden Ansprüche, **dadurch gekennzeichnet, dass** sie einen kosmetischen Bestandteil enthält, der unter den Antioxidantien, Parfums, Konservierungsmitteln, Fasern, Neutralisationsmitteln, grenzflächenaktiven Stoffen, Wachsen, Sonnenschutzfiltern, Vitaminen, Hydratisierungsmitteln, Selbstbräunungsmitteln und Wirkstoffen gegen Falten ausgewählt ist.

57. Zusammensetzung nach einem der vorhergehenden Ansprüche, **dadurch gekennzeichnet, dass** die Zusammensetzung als loser Puder, kompaktiertes Pulver, gepresstes Pulver oder pastöse Zusammensetzung vorliegt.

58. Zusammensetzung nach einem der vorhergehenden Ansprüche, **dadurch gekennzeichnet, dass** die Zusammensetzung eine feste Zusammensetzung ist.

59. Zusammensetzung nach einem der vorhergehenden Ansprüche, **dadurch gekennzeichnet, dass** die Zusammensetzung als Makeup, Lidschatten, Wangenrouge, Produkt gegen Augenringe, Produkt zum Schminken des Körpers, Produkt zum Schminken der Lippen vorliegt.

60. Verfahren zum Schminken der Haut oder der Lippen, das umfasst, eine Zusammensetzung nach einem der vorhergehenden Ansprüche auf die Haut oder die Lippen aufzutragen.
